(19) Europäisches Patentamt
European Patent Office
Office européen des brevets

(11) EP 1 759 005 B1

(12) **EUROPÄISCHE PATENTSCHRIFT**

(45) Veröffentlichungstag und Bekanntmachung des Hinweises auf die Patenterteilung:
**10.08.2011 Patentblatt 2011/32**

(21) Anmeldenummer: **05768621.4**

(22) Anmeldetag: **14.06.2005**

(51) Int Cl.:
***C12N 15/82*** *(2006.01)* ***C12N 15/53*** *(2006.01)*
***C12N 9/08*** *(2006.01)* ***A01H 5/10*** *(2006.01)*
***A01H 5/02*** *(2006.01)*

(86) Internationale Anmeldenummer:
**PCT/EP2005/006376**

(87) Internationale Veröffentlichungsnummer:
**WO 2006/000319 (05.01.2006 Gazette 2006/01)**

(54) **VERFAHREN ZUR ERHÖHUNG DER PATHOGENRESISTENZ IN TRANSGENEN PFLANZEN DURCH EXPRESSION EINER PEROXIDASE**

METHOD FOR INCREASING PATHOGEN-RESISTANCE IN TRANSGENIC PLANTS BY EXPRESSION OF PEROXIDASE

PROCEDES D'AMELIORATION DE LA RESISTANCE PATHOGENE DE PLANTES TRANSGENIQUES PAR L'EXPRESSION DE PEROXYDASE

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **24.06.2004 DE 102004030608**

(43) Veröffentlichungstag der Anmeldung:
**07.03.2007 Patentblatt 2007/10**

(73) Patentinhaber: **BASF Plant Science GmbH**
**67056 Ludwigshafen (DE)**

(72) Erfinder:
- **FRANK, Markus**
**67434 Neustadt (DE)**
- **SCHMIDT, Ralf-Michael**
**67489 Kirrweiler (DE)**
- **STAUDER, Sandra**
**67273 Weisenheim am Berg (DE)**

(74) Vertreter: **Patzelt, Andrea**
**BASF SE**
**GVX/A - C 6**
**Carl-Bosch-Straße 38**
**67056 Ludwigshafen (DE)**

(56) Entgegenhaltungen:
**EP-A- 1 249 493** **WO-A-97/41237**
**WO-A-02/070723** **DE-A1- 10 220 115**
**DE-A1- 10 220 115**

- **DATABASE EMBL EBI; 8. November 2002 (2002-11-08), ZHANG H ET AL: "Hordeum vulgare cDNA clone HS06P24 5-PRIME, mRNA sequence" XP002348183 Database accession no. CA002255**
- **KRISTENSEN BRIAN K ET AL: "Expression of a defence-related intercellular barley peroxidase in transgenic tobacco" PLANT SCIENCE (SHANNON), Bd. 122, Nr. 2, 1997, Seiten 173-182, XP002348177 ISSN: 0168-9452**
- **SHI W M ET AL: "Cloning of peroxisomal ascorbate peroxidase gene from barley and enhanced thermotolerance by overexpressing in Arabidopsis thaliana" GENE (AMSTERDAM), Bd. 273, Nr. 1, 25. Juli 2001 (2001-07-25), Seiten 23-27, XP002348178 ISSN: 0378-1119**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von transgenen Pflanzen und/oder Pflanzenzellen mit erhöhter Pilzpathogenresistenz, das dadurch gekennzeichnet ist, dass eine DNA-Sequenz, die für ein Protein mit der Aktivität einer Peroxidase kodiert, in die Pflanze eingeführt und dort exprimiert wird. Die vorliegende Erfindung betrifft ebenfalls die Verwendung von Nukleinsäuren, die für eine Peroxidase kodieren, zur Herstellung von transgenen Pflanzen bzw. Pflanzenzellen mit einer erhöhten pilzpathogenresistenz. Weiterhin betrifft die vorliegende Erfindung Nukleinsäuresequenzen, die für eine Peroxidase aus Gerste kodieren.

Pflanzenkrankheiten, die durch verschiedene Pathogene wie z.B. Viren, Bakterien und Pilzen verursacht werden, können beim Anbau von Kulturpflanzen zu erheblichen Ernteausfällen führen, was zum einen wirtschaftliche Folgen hat, aber auch die Sicherheit der menschlichen Ernährung bedroht. Seit dem letzten Jahrhundert werden zur Kontrolle von Pilzerkrankungen chemische Fungizide eingesetzt. Durch den Einsatz dieser Stoffe konnte zwar das Ausmaß von Pflanzenerkrankungen reduziert werden, es ist allerdings bis heute nicht auszuschließen, dass diese Verbindungen eine schädliche Wirkung auf Mensch, Tier und Umwelt ausüben. Um langfristig den Verbrauch von herkömmlichen Pflanzenschutzmitteln auf ein Minimum zu reduzieren, ist es daher von Bedeutung, die natürliche Pathogenabwehr von verschiedenen Pflanzen gegenüber unterschiedlichen Erregern zu untersuchen und sie sich durch gentechnologische Manipulation, z.B. durch das Einführen externer Resistenzgene oder durch die Manipulation der endogenen Genexpression in den Pflanzen, zur Herstellung von pathogenresistenten Pflanzen gezielt nutzbar zu machen.

Als Resistenz bezeichnet man die Fähigkeit einer Pflanze, Befall und Besiedlung durch einen Schaderreger zu verhindern oder zumindest zu begrenzen.

Bei den natürlicherweise auftretenden Resistenzen, mit denen sich die Pflanzen gegen die Besiedelung durch phytopathogene Organismen wehren, können verschiedene Mechanismen unterschieden werden. Diese spezifischen Wechselwirkungen zwischen Pathogen und Wirt entscheiden über den Infektionsverlauf (Schopfer und Brennicke (1999) Pflanzenphysiologie, Springer Verlag, Berlin-Heidelberg).

Im Zusammenhang mit der Rasse-spezifischen Resistenz, die auch Wirtsresistenz genannt wird, unterscheidet man zwischen der kompatiblen und der inkompatiblen Interaktion. Bei der kompatiblen Interaktion kommt es zur Wechselwirkung eines virulenten Pathogens mit einer anfälligen Pflanze. Das Pathogen überlebt und kann Vermehrungsstrukturen ausbilden, der Wirt dagegen stirbt ab. Von einer inkompatiblen Interaktion spricht man hingegen, wenn das Pathogen die Pflanze zwar infiziert, jedoch vor oder nach schwacher Symptomausprägung in seinem Wachstum gehemmt wird. In diesem Fall ist die Pflanze gegen den betreffenden Erreger resistent (Schopfer und Brennicke (1999) Pflanzenphysiologie, Springer Verlag, Heidelberg). Sowohl bei der kompatiblen als auch bei der inkompatiblen Interaktion findet eine Abwehrreaktion des Wirts gegenüber dem Pathogen statt.

Die genetische Grundlage für die kompatible bzw. inkompatible Wirt/Pathogen-Interaktion wurde durch die Gen-für-Gen-Hypothese beschrieben (Flor (1971) Annu. Rev. Phytopathology 9: 275 296). Die Vorraussetzung für die Rasse-spezifische Pathogenerkennung ist die direkte oder indirekte Interaktion des Produktes eines dominanten oder semi-dominanten Resistenzgens (*R*-Gen) der Pflanze mit einem Produkt, welches aus dem komplementären und dominanten Avirulenzgen (Avr-Gen) des Phytopathogens stammt (Keen (1992) Annu. Rev. Genet. 24: 447 - 463; Staskawicz et al. (1995) Science 268: 661 - 667). Wenn dem Krankheitserreger dagegen das komplementäre Avirulenzgen fehlt, kann es zum Ausbruch der Krankheit kommen. Das Gen-für-Gen-Modell hat sich für viele Rost-, Brand-, Echtesowie Falsche Mehltaupilzinfektionen bestätigt, ist jedoch nicht auf alle Wirt-Parasit-Interaktionen übertragbar.

In der Natur wird diese Rasse-spezifische Resistenz jedoch aufgrund der schnellen evolutionären Entwicklung der Pathogene häufig überwunden (Neu et al. (2003) American Cytopathol. Society, MPMI 16 Nr. 7: 626 - 633). Dagegen bietet die Nicht-Wirts-Resistenz einen starken, breiten und dauerhaften Schutz gegenüber Phytopathogenen. Unter der Nicht-Wirts-Resistenz versteht man das Phänomen, dass ein Pathogen in einer bestimmten Pflanzenspezies eine Krankheit auslösen kann, in einer anderen, genetisch ähnlichen Pflanzenspezies jedoch nicht (Heath (2002) Can. J. Plant Pathol. 24: 259 - 264).

Trotz dieser interessanten Eigenschaft sind die genetischen und molekularbiologischen Grundlagen für die Nicht-Wirts-Resistenz bisher nur wenig verstanden. Es gibt sowohl Anzeichen dafür, dass die Nicht-Wirts-Resistenz durch unspezifische Stoffe induziert wird als auch dafür, dass einzelne Pathogenproteine in der Art der Gen-für-Gen-Interaktion die Nicht-Wirts-Resistenzreaktion auslösen (Heath (1981) Phytopathology 71: 1121 - 1123; Heath (2001) Physiol. Mol. Plant Pathol. 58: 53 - 54; Kamoun et al. (1998) Plant Cell 10: 1413 - 1425; Lauge et al. (2000) Plant J. 23: 735 - 745; Whalen et al. (1988) Proc. Natl. Acad. Sci. USA 85: 6743 - 6747).

Ein weiterer Grund, weshalb es nur selten zur Besiedelung von Pflanzen durch Phytopathogene kommt, ist, dass das Pathogen aufgrund eines Mangels an Stoffen und Strukturen, die zum Infektionsverlauf benötigt werden, keine Infektionsstrukturen ausbilden kann. Außerdem können auch unspezifisch induzierte oder präformierte Resistenzbarrieren, die schon vor der Infektion durch ein Pathogen vorliegen, die Bildung von Infektionsstrukturen verhindern. Diese Resistenz wird als Basisresistenz oder Rasse-unspezifische Resistenz bezeichnet und durch passive und aktive Abwehrmechanismen aufrechterhalten.

Die unterschiedlichen Resistenzmechanismen, die für die Resistenz oder Empfänglichkeit einer Pflanzenart bzw. deren Kultivaren gegenüber bestimmten Pflanzenpathogenen verantwortlich sind, sollen beispielhaft für den Mehltauerreger (*Blumeria graminis*), der mehrere unterschiedliche Getreide befällt, dargestellt werden. Der Mehltaupilz gehört zur Gattung der *Ascomycetes* und befällt außer Weizen und Gerste auch Roggen, Hafer und zahlreiche Gräser. Die bei Gerste und Weizen verursachten Ertragsausfälle können in Einzelfällen bis zu 25% betragen. In der Regel liegen sie aber zwischen 5 und 15%. Zudem schafft der Mehltau auch Eintrittspforten für andere Krankheitserreger (Krebsschimmel, Spelzenbräune, Fusarium-Blattbefall usw.). Das auffälligste Krankheitssymptom einer Mehltauinfektion sind die weißen "Mehltaupusteln", die vorwiegend auf der Blattoberseite und den Blattscheiden auftreten. Der echte Mehltau, *Blumeria graminis,* ist obligat biotroph, d.h. er kann sich nur von lebender Substanz ernähren und nicht auf Nährmedium kultiviert werden. Stattdessen beeinflusst der Pilz den Wirtsmetabolismus derart, dass sein eigenes Wachstum gefördert wird (Ferrari et al. (2003) The Plant Journal 35: 193 - 205). Der Mehltaupilz befällt ausschließlich die Epidermiszellschicht von Gerstenblättern. Der Pilz dringt in die Pflanzenzelle mittels eines Penetrationspflocks ein, bei dem es sich um Konidien, d.h. asexuell gebildete Sporen handelt, mechanisch und enzymatisch durch die Zellwand. Der erfolgreiche Befall von Gerstenblättern ist erreicht, wenn sich das Haustorium, bei dem es sich um das pilzliche Versorgungsorgan handelt, gebildet hat. Die Haustorien entziehen der Pflanzenzelle die Nährstoffe, worauf die nicht-infizierten benachbarten Zellen diesen Nährstoffverlust der Zelle ausgleichen und sie dadurch am Leben halten. Durch den Nährstofffluss in die geschädigte Zelle sichert sich der Pilz für eine gewisse Zeit eine Überlebensgrundlage.

Der Mehltaupilz als Art umfasst mehrere Formae speciales abhängig davon, ob der jeweilige Mehltaupilz z.B. Weizen oder Gerste befällt. Im Falle des Befalls von Gerste handelt es sich um *Blumeria graminis f sp. hordei,* während es sich beim Befall von Weizen um *Blumeria graminis f sp. tritici* handelt. Darüber hinaus können innerhalb der verschiedenen Formae speciales unterschiedliche Rassen oder Pathotypen identifiziert werden, denen gegenüber unterschiedliche Kultivare der Wirtsarten eine unterschiedliche Resistenz aufweisen. Die jeweiligen Formae speciales befallen jeweils nur eine bestimmte Pflanzenart, nicht jedoch andere, nahe verwandte Pflanzen. Es handelt sich hierbei also um eine Nicht-Wirts-Resistenz der Pflanzen, die nicht von einer bestimmten forma specialis befallen werden.

Es können mehrere unterschiedliche genetische Mechanismen unterschieden werden, die der Gerste Resistenz gegenüber Mehltau verleihen. Die Rasse-spezifische Resistenz beruht auf Resistenzgenen (R-Gene) gegen den echten Gerstenmehltau, die nur gegen einzelne Isolate des Pilzes *Blumeria graminis f. sp. hordei* wirksam sind, weil jedes einzelne R-Gen, wie oben bereits erwähnt, nur jenes Isolat erkennt, welches ein komplementäres Avirulenzgen trägt (Collins et al. (2002) The Powdery Mildew Accomprehensive Treatise, American Phytopathological Society, Minnesota; Peterhänsel et al. (1997) Plant Cell 9: 1397 - 1409). Im Gegensatz dazu wird die breite, Rasse-unspezifische Resistenz gegenüber mehreren Mehltau-Isolaten durch ein einzelnes rezessives Gen kontrolliert, das als *Mlo*-Gen bezeichnet wurde (Schulze-Lefert und Vogel (2000) Trends in Plant Science 5: 343 - 348). Eine aktuelle Hypothese zur Wirkung des *Mlo*-Gens ist, dass das *Mlo*-Wildtypprotein ein negativer Regulator der Abwehrreaktion ist und daher das Fehlen des Proteins eine schnellere und stärkere Abwehrreaktion bewirkt, woraus eine effektive Pathogenabwehr resultiert (Collins et al. (2002), vide supra). Die *Mlo*-vermittelte Resistenz führt zur Bildung einer subzellulären Zellwandapposition, die als Papille bezeichnet wird und sich direkt unterhalb des pilzlichen Penetrationspflocks, dem so genannten Appressorium, bildet. Daher werden die Penetrationsversuche des Pilzes auf der Stufe der Papillenbildung gehemmt, d.h. es kommt nicht zur Ausbildung eines Haustoriums, was für die Nährstoffversorgung des Pilzes und damit für die Etablierung eines effizienten Befalls notwendig ist.

Im Gegensatz zur Rasse-unspezifischen Resistenz werden bei der Nicht-Wirts-Resistenz der Gerste gegen den echten Mehltau *Blumeria graminis f. sp. tritici (Bgt),* der Weizen, aber nicht Gerste befällt, ähnliche Abwehrmechanismen induziert wie bei einer Inokulation von Gerste mit einer avirulenten Form von *Blumeria graminis f sp. hordei (Bgh).* Da die Nicht-Wirts-Resistenz stark, breit und dauerhaft ist, ist es von großem Interesse, die Gene zu identifizieren, die für die Nicht-Wirts-Resistenz verantwortlich sind. Dadurch könnte die Nicht-Wirts-Resistenz der Gerste agronomisch auch für andere Wirts-Pathogensysteme ausgenutzt werden. Es besteht daher ein Bedarf an Pflanzen bzw. Pflanzenzellen, die Gene, die die Nicht-Wirts-Resistenz vermitteln, exprimieren und somit eine erhöhte Resistenz gegenüber pilzlichen Erregern wie z.B. Mehltau zeigen.

Es ist eine Aufgabe der vorliegenden Erfindung, transgene Pflanzen bzw. Pflanzenzellen zur Verfügung zu stellen, die eine erhöhte Resistenz gegenüber Pilzpathogenen aufweisen. Es ist weiterhin eine Aufgabe der vorliegenden Erfindung, Pflanzen bzw. Pflanzenzellen mit einer Nicht-Wirts-Resistenz gegenüber verschiedenen Pilzpathogenen wie z.B. Mehltau zur Verfügung zu stellen. Darüber hinaus ist es eine Aufgabe der vorliegenden Erfindung, Verfahren zur Verfügung zu stellen, die die Herstellung der oben genannten transgenen Pflanzen bzw. Pflanzenzellen mit einer erhöhten (Nicht-Wirts-)Resistenz gegenüber pflanzlichen Pilzpathogenen, wie z.B. Mehltau, ermöglichen

Weiterhin ist es eine Aufgabe der vorliegenden Erfindung, DNA-Sequenzen zur Verfügung zu stellen, mit denen Pflanzen identifiziert werden können, die eine Nicht-Wirts-Resistenz gegenüber Pilzpathogenen aufweisen.

Zur Lösung dieser und weiterer Aufgaben, wie sie sich aus der Beschreibung ergeben, dienen die Merkmale der unabhängigen Ansprüche.

Bevorzugte Ausführungsformen der Erfindung sind durch die Merkmale der Unteransprüche definiert.

Im Rahmen der vorliegenden Erfindung wurde herausgefunden, dass bei der Interaktion von Gerste mit *Blumeria graminis sp. tritici* spezifisch eine Peroxidase induziert wird, die an der Vermittlung der Nicht-Wirts-Resistenz gegenüber dieser Forma specialis des Mehltaus beteiligt ist.

Die genannten Aufgaben der vorliegenden Erfindung werden daher im Wesentlichen dadurch gelöst, dass ein Verfahren zur Herstellung von transgenen Pflanzen mit erhöhter Pilzpathogenresistenz zur Verfügung gestellt wird, das dadurch gekennzeichnet ist, dass eine DNA-Sequenz, die für ein Protein mit der Aktivität einer Peroxidase kodiert, in die Pflanze eingeführt und dort exprimiert wird.

In der vorliegenden Erfindung wurden durch Oligonucleotide Fingerprinting-Studien Gene identifiziert, die bei der Interaktion von Gerste mit *Bgt* im Vergleich zu der Interaktion von Gerste mit *Bgh* sowie zur Kontrolle transkriptionell aktiviert wurden. Dieses Genexpressionsmuster wurde in der Folge durch verschiedene molekularbiologische Methoden bestätigt und die Volllängensequenz des am stärksten induzierten Gens bestimmt. Dieses Gen wurde anschließend als Peroxidase identifiziert und als HvBgt1 bezeichnet. Die Expression der HvBgt1 steigt bei der Nicht-Wirts-Reaktion von Gerste mit *Bgt* schon nach 24 Stunden stark an, während in der Wirts-Reaktion (Gerste mit *Bgh*) über den gesamten Verlauf nur eine geringe Erhöhung der Expression zu erkennen ist (vgl. Tab. 5 und Abb. 1).

Peroxidasen sind Enzyme, durch die Wasserstoffperoxid als Akzeptor von Wasserstoff-Molekülen organischer Verbindungen zu Wasser reduziert wird. Den Peroxidasen kommt in Pflanzen eine wichtige Funktion bei der Synthese der Zellwand und bei der Entgiftung xenobiotischer Stoffe zu. Während der PathogenInfektion werden reaktive Sauerstoffspezies gebildet, die nachteilig für die Pflanze sein können. Es wird angenommen, dass die Peroxidasen diese reaktiven Sauerstoffspezies enzymatisch umsetzen und dadurch unschädlich machen. Peroxidasen aus Mais und deren verwendungen zur Herstellung von transgenen Pflanzen mit erhöhter Pilzpathogen resistenz sind in WO 02/07023 beschrieben. Gegenstand der vorliegenden Erfindung ist daher ein isoliertes Nukleinsäuremolekül, das eine Nukleinsäuresequenz ausgewählt aus der Gruppe bestehend aus:

i) DNA-Sequenzen umfassend Nukleotidsequenzen, die von SEQ ID No. 1 kodiert werden,
ii) DNA-Sequenzen umfassend Nukleotidsequenzen, die für ein Protein mit der in SEQ ID No. 2-angegebenen Aminosäuresequenz kodieren,
iii) DNA-Sequenzen, die über die gesamte sequenz zu der in SEQ ID No. 1 angegebenen Nukleotidsequenz eine Sequenzidentität von mindestens 80% aufweisen,

enthält, und für ein Protein mit der Aktivität einer Peroxidase kodiert.

Bevorzugt stammt die Nukleinsäuresequenz aus *Hordeum vulgare.*

Ein weiterer Gegenstand der vorliegenden Erfindung sind Proteine , die durch die vorhergehend genannten Nukleinsäuremoleküle kodiert werden.

Gegenstand der vorliegenden Erfindung ist auch ein Verfahren zur Herstellung von transgenen Pflanzen bzw. Pflanzenzellen mit erhöhter Pilzpathogenresistenz, das dadurch gekennzeichnet ist, dass eine DNA-Sequenz ausgewählt aus der Gruppe bestehend aus:

i) DNA-Sequenzen umfassend Nukleotidsequenzen, die von SEQ ID No. 1 oder kodiert werden,
ii) DNA-Sequenzen umfassend Nukleotidsequenzen, die für Proteine mit der in SEQ ID No. 2 angegebenen Aminosäuresequenz kodieren,
iii) DNA-Sequenzen, die über die gesamte Sequenz zu der in SEQ ID No. 1 angegebenen Nukleotidsequenz eine Sequenzidentität von mindestens 80% aufweisen,

die für ein Protein mit der Aktivität einer Peroxidase kodiert, in die Pflanze bzw. Pflanzenzelle eingeführt und dort exprimiert wird.

Ein weiterer Gegenstand der vorliegenden Erfindung sind rekombinante Nukleinsäuremoleküle, die in 5'-3'-Orientierung umfassen:

- regulatorische Sequenzen eines in Pflanzenzellen aktiven Promotors,
- operativ daran gebunden eine Nukleinsäuresequenz wie oben angegeben,
- ggf. operativ daran gebunden regulatorische Sequenzen, die in der Pflanzenzelle als Transkriptions-, Terminations- und/oder Polyadenylierungssignale dienen können.

Gegenstand der vorliegenden Erfindung sind ebenfalls transgene Pflanzen bzw. Pflanzenzellen, die nach einem der erfindungsgemäßen Verfahren hergestellt wurden und über im Vergleich zum Wildtyp erhöhte Pilzpathogenresistenz und einen höheren Peroxidase-Gehalt verfügen.

Ebenfalls Gegenstand der vorliegenden Erfindung ist die Verwendung der in der Erfindung beschriebenen Nukleinsäuresequenzen zur Herstellung von transgenen Pflanzen und Pflanzenzellen mit erhöhter Pilzpathogenresistenz.

Ein weiterer Gegenstand der Erfindung ist die Verwendung einer in der Erfindung beschriebenen Nukleinsäuresequenz zur Identifizierung von Pflanzen, die eine Nicht-Wirts-Resistenz gegenüber Pilzpathogenen zeigen.

Pilzpathogenresistenz" meint das Vermindern oder Abschwächen von Krankheitssymptomen einer Pflanze infolge eines Befalls durch ein Pilzpathogen. Die Symptome können vielfältiger Art sein, umfassen aber bevorzugt solche, die direkt oder indirekt zu einer Beeinträchtigung der Qualität der Pflanze, der Quantität des Ertrags, der Eignung zur Verwendung als Futter- oder Nahrungsmittel führen oder aber auch Aussaat, Anbau, Ernte oder Prozessierung des Ernteguts erschweren.

Unter dem Begriff "erhöhte Pilzpathogenresistenz" wird erfindungsgemäß verstanden, dass die erfindungsgemäßen transgenen Pflanzen bzw. Pflanzenzellen durch pilzliche Pathogene weniger stark und/oder weniger häufig befallen werden als nichttransformierte Wildtyp-Pflanzen bzw. Pflanzenzellen, die ansonsten gleich behandelt wurde (z.B. Klima- und Anbaubedingungen, Pathogenart, usw.). Bevorzugt ist der Befall mit Pathogenen mindestens um den Faktor 2, besonders bevorzugt mindestens um den Faktor 3, insbesondere bevorzugt mindestens um den Faktor 5 und am meisten bevorzugt mindestens um den Faktor 10 reduziert, was sich in einer Verminderung der Ausbildung von Krankheitssymptomen äußert. Eine Möglichkeit, den Pathogenbefall zu quantifizieren, bietet die Penetrationseffizienz (siehe Beispiel 9). Der Begriff "erhöhte Pathogenresistenz" beinhaltet dabei auch eine so genannte transiente Pathogenresistenz, d.h. dass die erfindungsgemäßen transgenen Pflanzen bzw. Pflanzenzellen nur für einen begrenzten Zeitraum eine gegenüber dem entsprechenden Wildtyp erhöhte Pathogenresistenz aufweisen.

Bei den Pilzpathogenen" kann es sich um jeden pilzlichen Erreger handeln, der die Wildtyppflanze normalerweise befällt, wie beispielsweise Mehltau. Es ist aber auch anzunehmen, dass die Überexpression einer erfindungsgemäß verwendeten Sequenz auch eine Resistenz gegen weitere Pathogene bewirkt.

Pilzpathogene oder pilz-ähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft, jedoch nicht einschränkend, seien die in Tabelle 1 genannten Pilzpathogene und die mit ihnen in Zusammenhang gebrachten Erkrankungen zu nennen.

Tabelle 1: Pflanzliche Pilzerkrankungen

| Erkrankung | Pathogen |
|---|---|
| Braunrost | Puccinia recondita |
| Gelbrost | P. striiformis |
| Echter Mehltau | Erysiphe graminis / Blumeria graminis |
| Spelzenbräune | Septoria nodorum |
| Blattdürre | Septoria tritici |
| Ährenfusariosen | Fusarium spp. |
| Halmbruchkrankheit | Pseudocercosporella herpotrichoides |
| Flugbrand | Ustilago spp. |
| Weizensteinbrand | Tilletia caries |
| Schwarzbeinigkeit | Gaeumannomyces graminis |
| Anthracnose leaf blight<br><br>Anthracnose stalk rot | Colletotrichum graminicola (teleomorph: Glomerella graminicola Politis); Glomerella<br>tucumanensis (anamorph: Glomerella falcatum Went) |
| Aspergillus ear and kernel rot | Aspergillus flavus |
| Banded leaf and sheath spot | Rhizoctonia solani Kuhn = Rhizoctonia |
| ("Wurzeltöter") | microsclerotia J. Matz (telomorph: Thanatephorus cucumeris) |
| Black bundle disease | Acremonium strictum W. Gams = Cephalosporium acremonium Auct. non Corda |
| Black kernel rot | Lasiodiplodia theobromae = Botryodiplodia theobromae |
| Borde blanco | Marasmiellus sp. |

(fortgesetzt)

| Erkrankung | Pathogen |
|---|---|
| Brown spot (black spot, stalk rot) | Physoderma maydis |
| Cephalosporium kernel rot | Acremonium strictum = Cephalosporium acremonium |
| Charcoal rot | Macrophomina phaseolina |
| Corticium ear rot | Thanatephorus cucumeris = Corticium sasakii |
| Curvularia leaf spot | Curvularia clavata, C. eragrostidis, = C. maculans (teleomorph: Cochliobolus eragrostidis), Curvularia inaequalis, C. intermedia (teleomorph: Cochliobolus intermedius), Curvularia lunata (teleomorph: Cochliobolus lunatus), Curvularia pallescens (teleomorph: Cochliobolus pallescens), Curvularia senegalensis, C. tuberculata (teleomorph: Cochliobolus tuberculatus) |
| Didymella leaf spot | Didymella exitalis |
| Diplodia ear rot and stalk rot | Diplodia frumenti (teleomorph: Botryosphaeria festucae) |
| Diplodia ear rot, stalk rot, seed rot and seedling blight | Diplodia maydis = Stenocarpella maydis |
| Diplodia leaf spot or streak | Stenocarpella macrospora = Diplodialeaf macrospora |
| Brown stripe downy mildew | Sclerophthora rayssiae var. zeae |
| Crazy top downy mildew | Sclerophthora macrospora = Sclerospora macrospora |
| Green ear downy mildew (graminicola downy mildew) | Sclerospora graminicola |
| Java downy mildew | Peronosclerospora maydis = Sclerospora maydis |
| Philippine downy mildew | Peronosclerospora philippinensis = Sclerospora philippinensis |
| Sorghum downy mildew | Peronosclerospora sorghi = Sclerospora sorghi |
| Spontaneum downy mildew | Peronosclerospora spontanea = Sclerospora spontanea |
| Sugarcane downy mildew | Peronosclerospora sacchari = Sclerospora sacchari |
| Dry ear rot (cob, kernel and stalk rot) | Nigrospora oryzae (teleomorph: Khuskia oryzae) |
| Ear rots, minor | Alternaria alternata = A. tenuis,<br>Aspergillus glaucus, A. niger,<br>Aspergillus spp., Botrytis cinerea (teleomorph: Botryotinia fuckeliana), Cunninghamella sp.,<br>Curvularia pallescens,<br>Doratomyces stemonitis = Cephalotrichum stemonitis,<br>Fusarium culmorum,<br>Gonatobotrys simplex,<br>Pithomyces maydicus,<br>Rhizopus microsporus Tiegh.,<br>R. stolonifer = R. nigricans,<br>Scopulariopsis brumptii |
| Ergot (horse's tooth) | Claviceps gigantea<br>(anamorph: Sphacelia sp.) |
| Eyespot | Aureobasidium zeae = Kabatiella zeae |
| Fusarium ear and stalk rot | Fusarium subglutinans = F. moniliforme var.subglutinans |

(fortgesetzt)

| Erkrankung | Pathogen |
|---|---|
| Fusarium kernel, root and stalk rot, seed rot and seedling blight | Fusarium moniliforme (teleomorph: Gibberella fujikuroi) |
| Fusarium stalk rot,<br>seedling root rot | Fusarium avenaceum<br>(teleomorph: Gibberella avenacea) |
| Gibberella ear and stalk rot<br>(Ähren- u. Stengelfäule) | Gibberella zeae<br>(anamorph: Fusarium graminearum) |
| Gray ear rot | Botryosphaeria zeae = Physalospora zeae<br>(anamorph: Macrophoma zeae) |
| Gray leaf spot (Cercospora leaf spot) | Cercospora sorghi = C. sorghi var. maydis, C. zeae-maydis |
| Helminthosporium root rot | Exserohilum pedicellatum = Helminthosporium pedicellatum (teleomorph: Setosphaeria pedicellata) |
| Hormodendrum ear rot (Cladosporium rot) | Cladosporium cladosporioides = Hormodendrum cladosporioides, C. herbarum (teleomorph: Mycosphaerella tassiana) |
| Hyalothyridium leaf spot | Hyalothyridium maydis |
| Late wilt | Cephalosporium maydis |
| Leaf spots, minor | Alternaria alternata,<br>Ascochyta maydis, A. tritici,<br>A. zeicola, Bipolaris victoriae = Helminthosporium victoriae<br>(teleomorph: Cochliobolus victoriae), C. sativus (anamorph: Bipolaris sorokiniana = H. sorokinianum = H. sativum), Epicoccum nigrum,<br>Exserohilum prolatum = Drechslera prolata<br>(teleomorph: Setosphaeria prolata)<br>Graphium penicillioides,<br>Leptosphaeria maydis, Leptothyrium zeae,<br>Ophiosphaerella herpotricha, (anamorph: Scolecosporiella sp.),<br>Paraphaeosphaeria michotii, Phoma sp., Septoria zeae, S. zeicola,<br>S. zeina |
| Northern corn leaf blight (white blast, crown stalk rot, stripe) | Setosphaeria turcica (anarnorph: Exserohilum turcicum = Helminthosporium turcicum) |
| Northern corn leaf spot Helminthosporium ear rot (race 1) | Cochliobolus carbonum (anamorph: Bipolaris zeicola = Helminthosporium carbonum) |
| Penicillium ear rot (blue eye, blue mold) | Penicillium spp., P. chrysogenum, P. expansum, P. oxalicum |
| Phaeocytostroma stalk rot and root rot | Phaeocytostroma ambiguum, = Phaeocytosporella zeae |
| Phaeosphaeria leaf spot | Phaeosphaeria maydis = Sphaerulina maydis |
| Physalospora ear rot (Botryosphaeria ear rot) | Botryosphaeria festucae = Physalospora zeicola (anamorph: Diplodia frumenti) |
| Purple leaf sheath | Hemiparasitic bacteria and fungi |
| Pyrenochaeta stalk rot and root | Phoma terrestris = |

(fortgesetzt)

| Erkrankung | Pathogen |
|---|---|
| rot | Pyrenochaeta terrestris |
| Pythium root rot | Pythium spp., P. arrhenomanes, P. graminicola |
| Pythium stalk rot | Pythium aphanidermatum = P. butleri L. |
| Red kernel disease (ear mold, leaf and seed rot) | Epicoccum nigrum |
| Rhizoctonia ear rot (sclerotial rot) | Rhizoctonia zeae (teleomorph: Waitea circinata) |
| Rhizoctonia root rot and stalk rot | Rhizoctonia solani, Rhizoctonia zeae |
| Root rots, minor | Alternaria alternata, Cercospora sorghi, Dictochaeta fertilis, Fusarium acuminatum (teleomorph: Gibberella acuminata), F. equiseti (teleomorph: G. intricans), F. oxysporum, F. pallidoroseum, F. poae, F. roseum, G. cyanogena, (anamorph: F. sulphureum), Microdochium bolleyi, Mucor sp., Periconia circinata, Phytophthora cactorum, P. drechsleri, P. nicotianae var. parasitica, Rhizopus arrhizus |
| Rostratum leaf spot (Helminthosporium leaf disease, ear and stalk rot) | Setosphaeria rostrata, (anamorph: Exserohilum rostratum = He/minthosporium rostratum) |
| Rust, common corn | Puccinia sorghi |
| Rust, southern corn | Puccinia polysora |
| Rust, tropical corn | Physopella pallescens, P. zeae = Angiopsora zeae |
| Sclerotium ear rot (southern blight) | Sclerotium rolfsii Sacc. (teleomorph: Athelia rolfsii) |
| Seed rot-seedling blight | Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae (anamorph: F. graminearum), Macrophomina phaseolina, Penicillium spp., Phomopsis sp., Pythium spp., Rhizoctonia solani, R. zeae, Sclerotium rolfsii, Spicaria sp. |
| Selenophoma leaf spot | Selenophoma sp. |
| Sheath rot | Gaeumannomyces graminis |
| Shuck rot | Myrothecium gramineum |
| Silage mold | Monascus purpureus, M ruber |
| Smut, common | Ustilago zeae = U. maydis |
| Smut, false | Ustilaginoidea virens |
| Smut, head | Sphacelotheca reiliana = Sporisorium holcisorghi |
| Southern corn leaf blight and stalk rot | Cochliobolus heterostrophus (anamorph: Bipolaris maydis = Helminthosporium maydis) |
| Southern leaf spot | Stenocarpella macrospora = Diplodia macrospora |

(fortgesetzt)

| Erkrankung | Pathogen |
|---|---|
| Stalk rots, minor | Cercospora sorghi, Fusarium episphaeria, F. merismoides, F. oxysporum Schlechtend, F. poae, F. roseum, F. solani (teleomorph: Nectria haematococca), F. tricinctum, Mariannaea elegans, Mucor sp., Rhopographus zeae, Spicaria sp. |
| Storage rots | Aspergillus spp., Penicillium spp. and other fungi |
| Tar spot | Phyllachora maydis |
| Trichoderma ear rot and root rot | Trichoderma viride = T. lignorum teleomorph: Hypocrea sp. |
| White ear rot, root and stalk rot | Stenocarpella maydis = Diplodia zeae |
| Yellow leaf blight | Ascochyta ischaemi, Phyllosticta maydis (teleomorph: Mycosphaerella zeae-maydis) |
| Zonate leaf spot | Gloeocercospora sorghi |

Besonders bevorzugt bewirkt die Peroxidase eine Resistenz gegen

- Plasmodiophoromycota wie Plasmodiophora brassicae (Kohlhernie, clubroot of crucifers), Spongospora subterranea (powdery scab of potato tubers), Polymyxa graminis (root disease of cereals and grasses);
- Oomycota wie Bremia lactucae (Falscher Mehltau an Salat), Peronospora (Falscher Mehltau) bei snapdragon (P. antirrhini), Zwiebel (P. destructor), Spinat (P. effusa), Sojabohne (P. manchurica), Tabak ("blue mold" = Blauschimmel; P. tabacina) Alfalfa und Klee (P. trifolium), Pseudoperonospora humuli (Falscher Mehltau an Hopfen), Plasmopara (Falscher Mehltau bei Trauben) (P. viticola) und Sonnenblume (P. halstedii), Sclerophtohra macrospora (Falscher Mehltau bei Cerealien und Gäsern), Pythium (seed rot, seedling damping-off, and root rot and all types of plants, z.B. Wurzelbrand an Beta-Rübe durch P. debaryanum), Phytophthora infestans (Kraut- und Knollenfäule bei Kartoffel, Braunfäule bei Tomate etc.), Albugo spec. (white rust on cruciferous plants);
- Ascomycota wie Microdochium nivale (Schneeschimmel an Roggen und Weizen), Fusarium graminearum, Fusarium culmorum (Ährenfäule v.a. bei Weizen), Fusarium oxysporum (Fusarium-Welke an Tomate), Blumeria graminis (Echter Mehltau an Gerste (f.sp. hordei) und Weizen (f.sp. tritici)), Erysiphe pisi (Erbsenmehltau), Nectria galligena (Obstbaumkrebs), Unicnula necator (Echter Mehltau der Weinrebe), Pseudopeziza tracheiphila (Roter Brenner der Weinrebe), Claviceps purpurea (Mutterkorn an z.B. Roggen und Gräsern), Gaeumannomyces graminis (Schwarzbeinigkeit an Weizen, Roggen u.a. Gräsern), Magnaporthe grisea (rice blast disease), Pyrenophora graminea (Streifenkrankheit an Gerste), Pyrenophora teres (Netzfleckenkrankheit an Gerste), Pyrenophora tritici-repentis (Blattfleckenkrankheit (Blattdürre) an Weizen), Venturia inaequalis (Apfelschorf), Sclerotinia sclerotium (Weißstengeligkeit, Rapskrebs), Pseudopeziza medicaginis (Klappenschorf an Luzerne, Weiß- und Rotklee);
- Basidiomyceten wie Typhula incarnata (Typhula-Fäule an Gerste, Roggen, Weizen), Ustilago maydis (Beulenbrand an Mais), Ustilago nuda (Flugbrand an Gerste), Ustilago tritici (Flugbrand an Weizen, Dinkel), Ustilago avenae (Flugbrand an Hafer), Rhizoctonia solani (Wurzeltöter an Kartoffeln), Sphacelotheca spp. (head smut of sorghum), Melampsora lini (rust of flax), Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer), Puccinia striiformis (Gelbrost an Weizen, Gerste, Roggen sowie zahlreichen Gräsern), Uromyces appendiculatus (Bohnenrost), Sclerotium rolfsii (root and stem rots of many plants);
- Deuteromyceten (Fungi imperfecti) wie Septoria nodorum (Spelzenbräune) an Weizen (Septoria tritici), Pseudocercosporella herpotrichoides (Halmbruchkrankheit an Weizen, Gerste, Roggen), Rynchosporium secalis (Blattfleckenkrankheit an Roggen und Gerste), Alternaria solani (Dürrfleckenkrankheit an Kartoffel, Tomate), Phoma betae (Wurzelbrand an Beta-Rübe), Cercospora beticola (Cercospora-Blattfleckenkrankheit an Beta-Rübe), (Alternaria brassicae (Rapsschwärze an Raps, Kohl u.a. Kreuzblütlern), Verticillium dahliae (Rapswelke und -stengelfäule), Colletotrichum lindemuthianum (Brennfleckenkrankheit an Bohne), Phoma lingam - Umfallkrankheit (Schwarzbeinigkeit an Kohl; Wurzelhals- oder Stengelfäule an Raps), Botrytis cinerea (Grauschimmel an Weinrebe, Erdbeere, Tomate, Hopfen etc.).

Am meisten bevorzugt sind die durch das erfindungsgemäße Verfahren hergestellten Pflanzen resistent gegen Phytophthora infestans (Kraut- und Knollenfäule, Braunfäule bei Tomate etc.), Microdochium nivale (vormals Fusarium nivale; Schneeschimmel an Roggen und Weizen), Fusarium graminearum, Fusarium culmorum (Ährenfäule an Weizen), Fusarium oxysporum (Fusarium-Welke an Tomate), Blumeria graminis (Echter Mehltau an Gerste (f. sp. hordei) und Weizen (f. sp. tritici)), Magnaporthe grisea (rice blast disease), Sclerotinia sclerotium (Weißstengeligkeit, Rapskrebs), Septoria nodorum und Septoria tritici (Spelzenbräune an Weizen), Alternaria brassicae (Rapsschwärze an Raps, Kohl u.a. Kreuzblütlern), Phoma lingam (Umfallkrankheit, Schwarzbeinigkeit an Kohl; Wurzelhals- oder Stengelfäule an Raps).

Bei einzelnen landwirtschaftlich besonders bedeutenden Sorten wirkt die erfindungsgemäß verwendete Peroxidase bevorzugt gegen die folgenden Pathogene:

Bei Gerste gegen die PilzPathogene Puccinia graminis f.sp. hordei (barley stem rust), Blumeria (Erysiphe) graminis f.sp. hordei (Barley Powdery Mildew).

Bei der Sojabohne gegen die Pilz Pathogene Phytophthora megasperma fsp.glycinea, Macrophomina phaseolina, Rhizoctonia solani, Sclerotinia sclerotiorum, Fusarium oxysporum, Diaporthe phaseolorum var. sojae (Phomopsis sojae), Diaporthe phaseolorum var. caulivora, Sclerotium rolfsii, Cercospora kikuchii, Cercospora sojina, Peronospora manshurica, Colletotrichum dematium (Colletotrichum truncatum), Corynespora cassiicola, Septoria glycines, Phyllosticta sojicola, Alternaria alternata, , Microsphaera diffussa, Fusarium semitectum, Phialophora gregata, Glomerella glycines, Phakopsorapachyrhizi, Pythium aphanidermatum, Pythium ultimum, Pythium debaryanum, Fusarium solani .

Bei Canola gegen die Pilz Pathogene Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata.

Bei Alfalfa gegen die Pilz-, Pathogene Pythium ultimum, Pythium irregulare, Pythium splendens, Pythium debaryanum, Pythium aphanidermatum, Phytophthora megasperma, Peronospora trifoliorum, Phoma medicaginis var. medicaginis, Cercospora medicaginis, Pseudopeziza medicaginis, Leptotrochila medicaginis, Fusarium, , Aphanomyces euteiches, Stemphylium herbarum, Stemphylium alfalfae.

Bei Weizen gegen die Pilzpathogene , Urocystis agropyri, , Alternaria alternata, Cladosporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis f.sp. tritici, Puccinia graminis f.sp. tritici, Puccinia recondita f.sp. tritici, Puccinia striiformis, Pyrenophora tritici-repentis, Septoria nodorum, Septoria tritici, Septoria avenae, Pseudocercosporella herpotrichoides, Rhizoctonia solani, Rhizoctonia cerealis, Gaeumannomyces graminis var. tritici, Pythium aphanidermatum, Pythium arrhenomanes, Pythium ultimum, Bipolaris sorokiniana, , Claviceps purpurea, Tilletia tritici, Tilletia laevis, Ustilago tritici, Tilletia indica, Rhizoctonia solani, Pythium arrhenomannes, Pythium gramicola, Pythium aphanidermatum, Puccinia graminis f.sp. tritici (Wheat stem rust), Blumeria (Erysiphe) graminis f.sp. tritici (Wheat Powdery Mildew). Bei der Sonnenblume gegen die Pilz Pathogene Plasmophora halstedii, Sclerotinia sclerotiorum, Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, , Cephalosporium acremonium, Phytophthora cryptogea, Albugo tragopogonis.

Bei Mais gegen die Pilz Pathogene Fusarium moniliforme var. subglutinans, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis 0, T (Cochliobolus heterostrophus), Helminthosporium carbonum I, II & III (Cochliobolus carbonum), Exserohilum turcicum I, II & III, Helminthosporium pedicellatum, Physoderma maydis, Phyllosticta maydis, Kabatiella maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Trichoderma viride, Claviceps sorghi, Diplodia macrospora, Sclerophthora macrospora, Peronosclerospora sorghi, Peronosclerospora philippinesis, Peronosclerospora maydis, Peronosclerospora sacchari, Spacelotheca reiliana, Physopella zeae, Cephalosporium maydis, Caphalosporium acremonium.

Bei Sorghum gegen die Pilz Pathogene Exserohilum turcicum, Colletotrichum graminicola (Glomerella graminicola), Cercospora sorghi, Gloeocercospora sorghi, Ascochyta sorghina, Puccinia purpurea, Macrophomina phaseolina, Perconia circinata, Fusarium monilifonne, Alternaria alternate, Bipolaris sorghicola, Helminthosporium sorghicola, Curvularia lunata, Phoma insidiosa, Ramulispora sorghi, Ramulispora sorghicola, Phyllachara sacchari, Sporisorium reilianum (Sphacelotheca reiliana), Sphacelotheca cruenta, Sporisorium sorghi, , Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola.

Unter einem "Wildtyp" wird erfindungsgemäß der entsprechende nicht genetisch veränderte Ausgangsorganismus verstanden.

Durch das erfindungsgemäße Verfahren wird eine Erhöhung des Peroxidase-Gehalts in einer transgenen Pflanze bzw. Pflanzenzelle herbeigeführt. Vorzugsweise beträgt diese Erhöhung des Gehalts mindestens 5%, bevorzugt mindestens 20%, ebenfalls bevorzugt mindestens 50%, besonders bevorzugt mindestens 100%, ebenfalls besonders bevorzugt mindestens den Faktor 5, insbesondere bevorzugt mindestens den Faktor 10, ebenfalls insbesondere bevorzugt mindestens den Faktor 50 und am meisten bevorzugt den Faktor 100.

Unter dem Begriff "Fragmente der DNA", wie er hier verwendet wird, versteht man Teilstücke der DNA, die für ein Protein kodieren, das die Aktivität einer Peroxidase besitzt, wobei die von den DNA-Teilstücken kodierten Proteine im wesentlichen die gleiche Peroxidaseaktivität aufweisen wie die von der vollständigen DNA-Sequenz kodierten Proteine und sich mit diesen Fragmenten die erfindungsgemäße Erhöhung der Pathogenresistenz in transgenen Pflanzen erzielen lässt.

Der Begriff "Fragmente des Proteins", wie er hier verwendet wird, bezeichnet Teilstücke des Proteins, das die Aktivität einer Peroxidase besitzt, wobei die Teilstücke des Proteins im wesentlichen die gleiche Peroxidaseaktivität aufweisen wie das Protein in seiner vollen Länge und sich mit diesen Fragmenten die erfindungsgemäße Erhöhung der Pathogenresistenz in transgenen Pflanzen erzielen lässt.

Unter im Wesentlichen gleicher enzymatischer Aktivität der im erfindungsgemäßen Verfahren verwendeten Peroxidase ist zu verstehen, dass die enzymatische Aktivität gegenüber den durch die Sequenz mit SEQ ID No. 1 und deren Derivate kodierten Enzymen im Vergleich noch mindestens 50%, bevorzugt mindestens 60%, besonders bevorzugt mindestens 70% und ganz besonders bevorzugt mindestens 80% und am meisten bevorzugt mindestens 90% beträgt. Peroxidasen mit im wesentlichen gleicher enzymatischer Aktivität sind damit ebenfalls geeignet, in transgenen Pflanzen eine erhöhte Pathogenresistenz zu bewirken.

Die Aktivität von Peroxidasen lässt sich durch einfache Verfahren, die dem Fachmann bekannt sind, bestimmen, z.B. über die weit verbreitete Guaiacol-Peroxidase-Aktivitätsmessung (Chance und Maehley (1955) Method Enzymol. 11: 764-775) oder die Syringaldazin-Aktivitätsmessung (Pandolfini et al. (1992) Plant Cell Environ. 15: 719-725).

Der Begriff "Nukleinsäure(molekül)", wie hier verwendet, umfasst in einer bevorzugten Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Peroxidasemolekül z.B. weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren. Bei allen hier erwähnten Nukleinsäuremolekülen kann es sich z.B. um RNA, DNA oder cDNA handeln.

Die im Verfahren verwendeten Nukleinsäuremoleküle, z.B. ein Nukleinsäuremolekül mit einer Nukleotidsequenz der SEQ ID No. 1 können unter Verwendung molekularbiologischer Standardtechniken und der hier bereit gestellten Sequenzinformation isoliert werden. Auch können mit Hilfe von Vergleichsalgorithmen, wie sie z.B. auf der NCBI-Homepage unter http://www.ncbi.nlm.nih.gov zu finden sind, beispielsweise eine homologe Sequenz oder homologe, konservierte Sequenzbereiche auf DNA- oder Aminosäureebene identifiziert werden. Wesentliche Teile dieser Sequenz oder die gesamte homologe Sequenz können als Hybridisierungssonde unter Verwendung von Standardhybridisierungstechniken (wie z.B. beschrieben in Sambrook et al., vide supra) zur Isolierung weiterer im Verfahren nützlicher Nukleinsäuresequenzen aus anderen Organismen durch das Screening von cDNA- und/oder genomischen Banken verwendet werden. Überdies lässt sich ein Nukleinsäuremolekül, umfassend eine vollständige Sequenz der SEQ ID No. 1 oder einen Teil davon, durch Polymerasekettenreaktion isolieren, wobei Oligonukleotidprimer auf der Basis der hier angegebenen Sequenzen oder von Teilen davon verwendet werden (z.B. kann ein Nukleinsäuremolekül, umfassend die vollständige Sequenz oder einen Teil davon, durch Polymerasekettenreaktion unter Verwendung von Oligonukleotidprimem isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18: 5294 - 5299) und daraus mittels reverser Transkriptase (z.B. Moloney-MLV-Reverse Transkriptase, erhältlich von Gibco/BRL, Bethesda, MD oder AMV-Reverse-Transkriptase, erhältlich von Seikagaku Amerika, Inc., St. Petersburg, FL) cDNA herstellen. Synthetische Oligonukleotidprimer zur Amplifizierung mittels Polymerasekettenreaktion lassen sich auf der Basis einer der in SEQ ID No. 1 dargestellten Sequenzen oder mit Hilfe der in SEQ ID No. 2 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern mittels Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Peroxidase-Nukleotidsequenz entsprechen, können durch Standardsyntheseverfahren, beispielsweise mit einem automatischen DNA-Synthesegerät, hergestellt werden. Die Peroxidase-Aktivität der durch diese Nukleinsäuresequenzen kodierten Proteine lässt sich dann durch die oben beschriebenen Enzymtests bestimmen. Mit den hier beschriebenen Verfahren lassen sich auch Pflanzen, die eine Nicht-Wirts-Resistenz zeigen, identifizieren, indem die Anwesenheit der erfindungsgemäß identifizierten Peroxidase nachgewiesen wird.

Der Begriff "Hybridisierung unter stringenten Bedingungen" bedeutet im Zusammenhang dieser Erfindung, dass die Hybridisierung *in vitro* unter Bedingungen durchgeführt wird, die stringent genug sind, um eine spezifische Hybridisierung zu gewährleisten. Stringente *in vitro*-Hybridisierungsbedingungen sind dem Fachmann bekannt und können der Literatur entnommen werden (z.B. Sambrook und Russell (2001) Molecular Cloning: A Laboratory Manual, 3. Auflage, Cold Spring Harbour Laboratory Press, Cold Spring Harbour, NY). Der Begriff "spezifische Hybridisierung" bezieht sich auf den Umstand, dass ein Molekül unter stringenten Bedingungen präferentiell an eine bestimmte Nukleinsäuresequenz, die Zielsequenz, bindet, wenn diese Teil einer komplexen Mischung von z.B. DNA- oder RNA-Molekülen ist, aber nicht oder zumindest wesentlich reduziert an andere Sequenzen.

Stringente Bedingungen sind von den Umständen abhängig. Längere Sequenzen hybridisieren spezifisch bei höheren Temperaturen. Generell werden stringente Bedingungen so ausgewählt, dass die Hybridisierungstemperatur circa 5˚C unter dem Schmelzpunkt ($T_m$) für die spezifische Sequenz bei einer definierten Ionenstärke und einem definierten pH liegt. Die $T_m$ ist die Temperatur (bei einem definierten pH-Wert, einer definierten Ionenstärke und einer definierten Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionenkonzentration (oder Konzentration eines anderen Salzes) bei einem pH zwischen 7,0 und 8,3 und die Temperatur mindestens 30˚C für kurze Moleküle (d.h. z.B. 10 bis 50 Nukleotide) beträgt. Zusätzlich können stringente Bedingungen die Zugabe von Stoffen wie z.B. Formamid, das die Hybride destabilisiert, umfassen. Bei einer Hybridisierung unter stringenten Bedingungen, wie hier verwendet, bleiben gewöhnlich Nukleotidsequenzen, die mindestens 60% homolog zueinander sind, aneinander hybridisiert. Vorzugsweise sind die stringenten Bedingungen derart gewählt, dass Sequenzen, die mindestens etwa 65%, bevorzugt mindestens etwa 70% und besonders bevorzugt mindestens etwa 75% oder stärker zueinander homolog sind, gewöhnlich aneinander hybridisiert bleiben. Ein bevorzugtes, nicht-einschränkendes Beispiel für stringente Hybridisierungsbedingungen sind Hybridisierungen in 6 x Natriumchlorid/Natriumcitrat (SSC) bei etwa 45˚C, gefolgt von einem oder mehreren Waschschritten in 0,2 x SSC, 0,1% SDS bei 50 bis 65˚C. Die Temperatur schwankt beispielsweise unter Standardhybridisierungsbedingungen je nach dem Typ der Nukleinsäure zwischen 42˚C und 58˚C in wässrigem Puffer mit einer Konzentration von 0,1 bis 5 x SSC (pH 7,2).

Falls organisches Lösungsmittel, z.B. 50% Formamid, im oben genannten Puffer vorliegt, liegt die Temperatur unter Standardbedingungen bei etwa 42˚C. Vorzugsweise sind die Hybridisierungsbedingungen für DNA:DNA-Hybride zum Beispiel 0,1 x SSC und 20˚C bis 45˚C, vorzugsweise 30˚C bis 45˚C. Vorzugsweise sind die Hybridisierungsbedingungen für DNA:RNA-Hybride zum Beispiel 0,1 x SSC und 30˚C bis 55˚C, vorzugsweise zwischen 45˚C bis 55˚C. Die vorstehend genannten Hybridisierungstemperaturen sind beispielsweise für eine Nukleinsäure mit etwa 100 Basenpaaren Länge und einem G/C-Gehalt von 50% in Abwesenheit von Formamid bestimmt. Der Fachmann weiß, wie die erforderlichen Hybridisierungsbedingungen anhand von Lehrbüchern, wie den vorstehend erwähnten oder den folgenden Lehrbüchern Current Protocols in Molecular Biology, John Wiley & Sons, N.Y. (1989), Hames und Higgins (Hrsgb.) 1985, Nucleic Acids Hybridization: A Practical Approach, IRL Press at Oxford University Press, Oxford; Brown (Hrsgb.) 1991, Essential Molecular Biology: A Practical Approach, IRL Press at Oxford University Press, Oxford, bestimmt werden können.

Der Begriff "Sequenzidentität" im Sinne der Erfindung bezeichnet eine Identität, also gleiche Nukleotide in gleicher 5'-3'-Reihenfolge, über die gesamte Sequenz zu der in SEQ ID No. 1 angegebenen Nukleinsäuresequenz von mindestens 80%, bevorzugt von mindestens 85%, besonders bevorzugt von mindestens 90% und am meisten bevorzugt von mindestens 95%.

Die Sequenzidentität wird über eine Reihe von Programmen, die auf verschiedenen Algorithmen beruhen, bestimmt. Dabei liefern die Algorithmen von Needleman und Wunsch oder Smith und Waterman besonders zuverlässige Ergebnisse. Für die Sequenzvergleiche wurde das Programm PileUp (Feng und Doolittle (1987) J. Mol. Evolution 25: 351- 360; Higgins et al. (1989) CABIOS 5: 151 - 153) oder die Programme Gap und Best Fit (Needleman und Wunsch (1970) J. Mol. Biol. 48: 443 - 453 und Smith und Waterman (1981) Adv. Appl. Math. 2: 482 - 489) verwendet, die im GCG-Software-Paket (Genetics Computer Group, 575 Science Drive, Madison, Wisconsin, USA) enthalten sind.

Die oben in Prozent angegebenen Sequenzidentitätswerte wurden mit dem Programm GAP über den gesamten Sequenzbereich mit folgenden Einstellungen ermittelt: Gap Weight: 50, Length Weight: 3, Average Match: 10000 und Average Mismatch: 0,000.

Diese Einstellungen wurden, falls nicht anders angegeben, als Standardeinstellungen für Sequenzvergleiche verwendet.

Nukleinsäuresequenzen, die von den in SEQ ID No. 1 angegebenen Nukleinsäuresequenzen abweichen, können z.B. durch Einbringen einer oder mehrerer Nukleotidsubstitutionen, -additionen oder -deletionen in eine Nukleotidsequenz der SEQ ID No. 1 erzeugt werden, so dass Proteine entstehen, in die gegenüber der in SEQ ID No. 2 angegebenen Sequenz eine oder mehrere Aminosäuresubstitutionen, -additionen oder -deletionen eingebracht wurden. Mutationen können in eine der Sequenzen der SEQ ID No. 1 durch Standardtechniken, wie z.B. stellenspezifische Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste, also an Aminosäureresten, die die enzymatische Aktivität der Peroxidase nicht beeinflussen, hergestellt. Bei einer "konservativen Aminosäuresubstitution" wird ein Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure und Glutaminsäure), ungeladenen polaren Seitenketten (z.B. Glycin, Asparagin, Glutamin, Serin, Threonin, Tyrosin, Cystein), unpolaren Seitenketten (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan). Ein vorhergesagter nicht-essentieller Aminosäurerest in der erfindungsgemäß verwendeten Peroxidase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der für die Peroxidase kodierenden Sequenz eingebracht werden, z.B. durch Sättigungsmutagenese, und die resultierenden Mutanten können nach der Peroxidase-Aktivität durchmustert werden, indem das kodierte Protein rekombinant exprimiert wird,um Mutanten zu identifizieren, die die Peroxidase-Aktivität beibehalten haben. Die Peroxidase-Aktivität des Proteins kann z.B. unter Verwendung der hier beschriebenen Tests bestimmt werden.

"Transgen" bzw. "rekombinant" bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassetten oder Vektoren alle solche durch gentechnische Methoden zustande gekommenen Konstruktionen, in denen sich entweder

a) die erfindungsgemäße Nukleinsäuresequenz, oder
b) eine mit der erfindungsgemäßen Nukleinsäuresequenz funktionell verknüpfte genetische Kontrollsequenz, zum Beispiel ein Promotor, oder
c) a) und b)

nicht in ihrer natürlichen, genetischen Umgebung befindet oder durch gentechnische Methoden modifiziert wurde, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der Peroxidase mit den entsprechenden Peroxidasegenen - wird zu einer transgenen Expressionskassette, wenn diese durch nicht-natürliche, synthetische ("künstliche") Verfahren wie beispielsweise eine Mutagenisierung geändert wird. Entsprechende Verfahren sind beispielsweise beschrieben in US 5,565,350 oder WO 00/15815.

Unter einer transgenen Pflanze bzw. Pflanzenzelle ist wie oben ausgeführt zu verstehen, dass sich die im Verfahren verwendeten Nukleinsäuren nicht an ihrer natürlichen Stelle im Genom der Pflanze bzw. Pflanzenzelle befinden, wobei die Nukleinsäuren homolog oder heterolog exprimiert werden können. Transgen bedeutet aber auch, dass die erfindungsgemäßen Nukleinsäuren zwar an ihrem natürlichen Platz im Genom eines Organismus sind, dass jedoch die Sequenz gegenüber der natürlichen Sequenz verändert wurde und/oder dass die Regulationssequenzen der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor.

Für den Fachmann ist klar, dass die für die Herstellung der transgenen Pflanze bzw. Pflanzenzelle verwendete Nukleinsäuresequenz, die für eine Peroxidase kodiert, ggf. an die Organismus-spezifische Kodonverwendung angepasst werden muss. Die Kodonverwendung lässt sich anhand von Computerauswertungen anderer bekannter Gene des gewählten Organismus bestimmen.

Bei einem bevorzugten erfindungsgemäßen Verfahren zur Herstellung von transgenen Pflanzen bzw. Pflanzenzellen mit erhöhter Pilzpathogenresistenz wird eine Nukleinsäuresequenz, die für eine erfindungsgemäße Peroxidase kodiert, auf eine Pflanze bzw. Pflanzenzelle übertragen. Diese Übertragung führt zu einer Erhöhung der Expression bzw. der Aktivität der Peroxidase im Vergleich zur Wildtyppflanze bzw. pflanzenzelle und entsprechend zu einer Erhöhung der Pilzathogenresistenz in den transgenen Pflanzen bzw. Pflanzenzellen.

Ein solches Verfahren umfasst erfindungsgemäß typischerweise die folgenden Schritte:

a) Herstellen eines Vektors, umfassend folgende Nukleinsäuresequenzen in 5'-3'-Orientierung:

- regulatorische Sequenzen eines in Pflanzenzellen aktiven Promotors,

- operativ damit verknüpft eine DNA-Sequenz, die für eine Peroxidase gemäß der vorliegenden Erfindung kodiert,
- operativ daran gebunden regulatorische Sequenzen, die in der Pflanzenzelle als Transkriptions-, Terminations- und/oder Polyadenylierungssignale dienen können,

b) Übertragen des Vektors aus Schritt a) auf eine Pflanzenzelle und Integration in das pflanzliche Genom; und

c) ggf. Regenerieren intakter Pflanzen aus den transformierten Pflanzenzellen.

Die im Verfahren verwendeten Nukleinsäuren können nach Einbringung in eine Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Expression der Peroxidase durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist.

Unter Erhöhung der Genexpression einer Nukleinsäure, die für eine Peroxidase kodiert, wird erfindungsgemäß auch die Manipulation der Expression der Pflanzeneigenen endogenen Peroxidase verstanden. Dies kann beispielsweise durch Veränderung der Promotor-DNA-Sequenz für Peroxidase-kodierende Gene erreicht werden. Eine solche Veränderung, die eine veränderte, vorzugsweise erhöhte Expressionsrate des endogenen Peroxidase-Gens zur Folge hat, kann durch Deletion oder Insertion von DNA-Sequenzen erfolgen. Eine Veränderung der Promotor-Sequenz von endogenen Peroxidase-Genen führt in der Regel zu einer Veränderung der exprimierten Menge des Peroxidase-Gens und damit auch zu einer Änderung der in der Zelle bzw. den Pflanzen nachweisbaren Peroxidase-Aktivität.

Eine weitere Möglichkeit zur Erhöhung der Aktivität und des Gehalts der endogenen Peroxidase besteht darin, Transkriptionsfaktoren, die in die Transkription der endogenen Peroxidase-Gene involviert sind, z.B. durch Überexpression hochzuregulieren. Die Maßnahmen zur Überexpression von Transkriptionsfaktoren sind dem Fachmann bekannt und werden ebenfalls im Rahmen der vorliegenden Erfindung für Peroxidasen offenbart.

Des Weiteren kann eine erhöhte Expression eines endogenen Peroxidase-Gens dadurch erzielt werden, dass ein im nicht transformierten Organismus nicht vorkommendes Regulatorprotein mit dem Promotor dieser Gene in Wechselwirkung tritt. Solch ein Regulator kann ein chimäres Protein darstellen, welches aus einer DNA-Bindedomäne und einer Transkriptionsaktivator-Domäne besteht, wie beispielsweise in WO 96/06166 beschrieben.

Die rekombinanten Nukleinsäuremoleküle, die zur Expression der Peroxidase verwendet werden, umfassen neben der zu übertragenden Nukleinsäuresequenz für die Peroxidase weitere regulatorische Elemente. Welche konkreten regulatorischen Elemente diese Vektoren enthalten müssen, hängt dabei jeweils von dem Verfahren ab, in dem diese Vektoren verwendet werden sollen. Über welche regulatorischen Elemente und auch andere Elemente diese Vektoren verfügen müssen, ist dem Fachmann, der mit den verschiedenen oben erwähnten Verfahren zum Herstellen von transgenen Pflanzen, in denen die Expression eines Proteins gesteigert wird, vertraut ist, bekannt.

Typischerweise handelt es sich bei den regulatorischen Elementen, die in den Vektoren enthalten sind, um solche, die die Transkription und, falls erwünscht, Translation in der Pflanzenzelle gewährleisten. Dies kann beispielsweise je nach ausgewählter Pflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und so die Expression der Nukleinsäure regulieren. Zusätzlich zu diesen neuen Regulationssequenzen oder anstelle dieser Sequenzen kann die natürliche Regulation der Sequenzen vor den eigentlichen Strukturgenen noch vorhanden sein und ggf. genetisch verändert worden sein, so dass die natürliche Regulation ausgeschaltet und die Expression der Gene erhöht wird. Das rekombinante Nukleinsäuremolekül kann aber auch einfacher aufgebaut sein, d.h. es sind keine zusätzlichen Regulationssignale vor die Nukleinsäuresequenz inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen auch allein vor das natürliche Gen zur Steigerung der Aktivität gebracht werden. Das Genkonstrukt kann außerdem vorteilhafterweise auch eine oder mehrere so genannte "Enhancer"-Sequenzen funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren.

Es ist im Prinzip möglich, alle natürlichen Promotoren mit ihren Regulationssequenzen, für das erfindungsgemäße Verfahren zu verwenden. Es ist jedoch auch möglich und vorteilhaft, zusätzlich oder alleine synthetische Promotoren zu verwenden.

Bei den Promotoren kann es sich sowohl um konstitutive, als auch um induzierbare oder gewebe- bzw. entwicklungsspezifische Promotoren handeln. Die Auswahl des Promotors wie auch anderer regulatorischer Sequenzen bestimmt dabei das räumliche und zeitliche Expressionsmuster und damit die Expresson der Peroxidase in transgenen Pflanzen.

Konstitutive Promotoren schließen ein z.B. den Kern-Promotor des Rsyn7-Promotors und andere konstitutive Promotoren, die in WO 99/43838 und US-Patent Nr. 6,072,050 beschrieben sind; den CaMV 35S- (Odell et al. (1985) Nature 313: 810 - 812); den Actin- (McElroy et al. (1990) Plant Cell 2:163 -171); den Ubiquitin-(Christensen et al. (1989) Plant Mol. Biol. 12: 619 - 632 und Christensen et al. (1992) Plant Mol. Biol. 18: 675 - 689); den pEMU- (Last et al. (1991) Theor. Appl. Genet. 81: 581 - 588); den MAS- (Velten et al. (1984) EMBO J. 3: 2723 - 2730); den ALS-Promotor (US - Patent 5,659,026) und ähnliche. Bei Verwendung eines konstitutiven Promotors kann eine zell- bzw. gewebespezifische Expression auch dadurch erreicht werden, dass die Genexpression in den Zellen bzw. Geweben, in denen sie nicht erwünscht ist, gehemmt wird, beispielsweise durch Ausprägung von Antikörpern, die das Genprodukt binden und somit seine Aktivität unterbinden, oder durch geeignete Inhibitoren, die in diesen Zellen wirken.

Bevorzugt wird das Peroxidase-Gen von einem induzierbaren Promotor exprimiert, und besonders bevorzugt von einem Pathogen-induzierbaren Promotor. Solche Promotoren schließen die von Pathogen-verwandten Proteinen (PR-Proteinen) ein, die nach der Infektion mit einem Pathogen induziert werden, z.B. PR-Proteine, SAR-Proteine, beta-1,3-Glucanase, Chitinase, usw. (siehe z.B. Redolfi et al. (1983) Neth. J. Plant Pathol. 89: 245 - 254; Uknes et al. (1992) Plant Cell 4: 645 - 656; und Van Loon (1985) Plant Mol. Virol. 4: 111-116).

Von besonderem Interesse sind auch Promotoren, die örtlich an oder nahe der Stelle der Pathogeninfektion exprimiert werden, wie z.B. die von Marineau et al. (1987) Plant Mol. Biol. 9: 335 - 342; Matton et al. (1989) Molecular Plant-Microbe Interactions 2: 325 - 331; Somsisch et al. (1986) Proc. Natl. Acad. Sci. USA 83: 2427 - 2430; Somsisch et al. (1988) Mol. Gen. Genet. 2: 93 - 98; Yang (1996) Proc. Natl. Acad. Sci. USA 93: 14972 -14977; Chen et al. (1996) Plant J. 10: 955 - 966; Zhang et al. (1994) Proc. Natl. Acad. Sci. USA 91: 2507 - 2511; Warner et al. (1993) Plant J. 3: 191 - 201; Siebertz et al. (1989) Plant Cell 1: 961 - 968 beschriebenen.

Außerdem sind auch wundinduzierbare Promotoren zur Verwendung im Verfahren der vorliegenden Erfindung bevorzugt, da Pathogene häufig durch Wunden eintreten. Solche wundinduzierbaren Promotoren schließen ein den des Proteinase-Inhibitors (pin II) aus Kartoffel (Ryan (1990) Ann. Rev. Phytopathol. 28: 425 - 449; Duan et al. (1996) Nature Biotechnology 14: 494 - 498); wun1 und wun2 (US-Patent Nr. 5,428,148); win1 und win2 (Stanford et al. (1989) Mol. Gen. Genet. 215: 200 - 208); Systemin (McGurl et al. (1992) Science 225: 1570 - 1573); WIP1 (Rohmeier et al. (1993) Plant Mol. Biol. 22: 783 - 792; Eckelkamp et al. (1993) FEBS Letters 323: 73 - 76); des MPI-Gens (Corderok et al. (1994) Plant J. 6 (2): 141- 150); der FGAM-Synthase (Vaghchhipawala et al. (2004) Genome 47 (2): 404 - 413; prxC2 (Kaothien et al. (2002) Plant Mol. Biol. 49 (6): 591- 599); poxA (Ito et al. (2000) Plant Science 155 (1): 85 - 100); FAD7 (Nishiuchi et al. (1999) Plant Physiol. 121 (4): 1239-1246); TR2' (WO 03/093483).

Chemisch regulierte Promotoren können verwendet werden, um die Expression eines Gens in einer Pflanze durch die Anwendung eines exogenen chemischen Regulators zu regulieren (siehe eine Übersicht in Gatz (1997) Annu. Rev. Plant Physiol. Plant Mol. Biol., 48: 89-108). Chemisch induzierbare Promotoren eignen sich besonders, wenn gewünscht wird, dass die Genexpression auf zeitspezifische Weise erfolgt. Beispiele dafür schließen ein den In2-2-Promotor aus Mais, der durch Benzolsulfonamid aktiviert wird, den GST-Promotor aus Mais, der durch hydrophobe elektrophile Verbindungen induziert wird, und den PR-1a-Promotor aus Tabak, der durch Salicylsäure aktiviert wird. Andere chemisch regulierbare Promotoren schließen ein Steroid-responsive Promotoren (siehe z.B. den Glucocorticoid-induzierbaren Promotor in Schena et al. (1991) Proc. Natl. Acad. Sci. USA 88: 10421 -10425 und McNellis et al. (1998) Plant J. 14 (2): 247 - 257), Ethanol-induzierbare und Tetracyclin-induzierbare Promotoren (siehe z.B. Gatz et al. (1991) Mol. Gen. Genet. 227: 229 - 237; US-Patent Nrn. 5,814,618 und 5,789,156).

Der Fachmann weiß, dass die Verwendung von induzierbaren Promotoren die Herstellung von Pflanzen bzw. Pflanzenzellen erlaubt, die die erfindungsgemäßen Sequenzen nur transient exprimieren. Eine solche transiente Expression erlaubt die Herstellung von Pflanzen, die eine nur transiente Pathogenresistenz zeigen. Solch eine transiente Resistenz kann z.B. dann erwünscht sein, wenn die Gefahr einer Pathogenkontamination droht und die Pflanzen deswegen nur für einen bestimmten Zeitraum resistent gegen das Pathogen sein müssen. Weitere Situationen, in denen eine transiente Resistenz wünschenswert ist, sind dem Fachmann bekannt. Dem Fachmann ist darüber hinaus auch bekannt, dass er auch durch die Verwendung nicht stabil in Pflanzenzellen replizierender Vektoren, die die entsprechenden Sequenzen zur Expression der Peroxidase tragen, eine transiente Expression und damit eine transiente Resistenz erzielen kann.

Außerdem können auch Gewebe-spezifische Promotoren verwendet werden, um eine erhöhte Peroxidase-Expression innerhalb eines bestimmten Pflanzengewebes zu erzielen. Geeignete Gewebe-spezifische Promotoren sind z.B. solche, die Blattspezifische Expression gewährleisten. Zu diesen gehören solche, die in Yamamoto et al. (1997) Plant J. 1 (2): 255 - 265; Kwon et al. (1994) Plant Physiol 105: 357-367; Yamamoto et al. (1994) Plant Cell Physiol. 35 (5): 773-778; Gotor et al. (1993) Plant J. 3: 509-518; Orozco et al. (1993) Plant Mol. Biol. 23 (6): 1129-1138: Matsuoka et al. (1993) Proc. Natl. Acad. Sci. USA 90 (20): 9586-9590; Stockhaus et al. (1987) Proc. Natl. Acad. G. USA 84: 7943 - 7947; Stockhaus et al. (1989) EMBO J. 8: 2445 - 2451 beschrieben sind.

Insbesondere bevorzugt ist es auch, Epidermis-spezifische Promotoren zu verwenden, da die Epidermis als Abschlussgewebe der oberirdischen Organe höherer Pflanzen unter anderem die Aufgabe hat, das Eindringen von Pathogenen in die Pflanze zu verhindern. Zu den geeigneten epidermis-spezifischen Promotoren gehört u.a. der Promotor des CER6- (CUT1-) Gens aus *Arabidopsis* (Hooker et al. (2002) Plant Physiol. 129 (4): 1568 -1580 und Kunst et al. (2000) Biochem. Soc. Trans. 28 (6): 651 - 654).

Weiterhin bevorzugte Promotoren sind solche, die insbesondere in Früchten aktiv sind. Hierzu zählen beispielsweise der Promotor eines Polygalacturonase-Gens, z.B. aus Tomate (Nicholass et al. (1995) Plant Mol. Biol. 28:423-435), der Promotor einer ACC-Oxidase, z.B. aus Apfel (Atkinson et al. (1998) Plant Mol. Biol. 38:449-460) oder der 2A11-Promotor aus Tomate (van Haaren et al. (1991) Plant Mol. Biol. 17:615-630).

Ebenfalls bevorzugt sind Mesophyll-spezifische Promotoren wie etwa der rbcs-Promotor aus Reis oder Tomate (Kyozuka et al. (1993) Plant Physiol. 102 (3): 991-1000), der PPCZm1-Promotor aus Mais (Kausch et al. (2001) Plant Mol Biol. 45 (1): 1-15), der CAB2-Promotor aus Arabidopsis (Thain et al. (2002) Plant Physiol. 130: 102-110) oder der AldP-Promotor aus Reis (Kagaya et al. (1995) Mol Gen Genet. 248 (6): 668-674).

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien z. B. weitere epidermis-spezifische regulatorische Nukleinsäureelemente identifizieren. Dabei wird z.B. in einem ersten Schritt das gewünschte Gewebe aus dem gewünschten Organismus, aus dem die regulatorischen Sequenzen isoliert werden sollen, isoliert und daraus die gesamte poly(A)$^+$-RNA isoliert und eine cDNA-Bank angelegt. In einem zweiten Schritt werden mit Hilfe von cDNA-Klonen, die auf poly(A)$^+$-RNA-Molekülen aus einem anderen Gewebe basieren, aus der ersten Bank mittels Hybridisierung diejenigen Klone identifiziert, deren korrespondierende poly(A)$^+$-RNA-Moleküle lediglich im gewünschten Gewebe akkumulieren. Anschließend werden mit Hilfe dieser so identifizierten cDNAs Promotoren isoliert, die über Gewebe-spezifische regulatorische Elemente verfügen. Dem Fachmann stehen darüber hinaus weitere auf PCR basierende Methoden für die Isolierung geeigneter Gewebe-spezifischer Promotoren zur Verfügung.

Die erfindungsgemäßen Vektoren können als regulatorische Elemente zusätzlich auch z.B. Enhancer-Elemente umfassen, ferner können sie Resistenzgene, Replikationssignale und weitere DNA-Bereiche enthalten, die eine Propagation der Vektoren in Bakterien wie z.B. *E. coli* ermöglichen. Die regulatorischen Elemente umfassen auch Sequenzen, die eine Stabilisierung der Vektoren in den Wirtszellen bewirken. Insbesondere umfassen solche regulatorischen Elemente Sequenzen, die eine stabile Integration des Vektors in das Wirtsgenom der Pflanze oder eine autonome Replikation des Vektors in den Pflanzenzellen ermöglichen. Solche regulatorischen Elemente sind dem Fachmann bekannt.

Bei den so genannten Terminationssequenzen handelt es sich um Sequenzen, die sicherstellen, dass die Transkription bzw. die Translation ordnungsgemäß beendet wird. Sollen die übertragenen Nukleinsäuren translatiert werden, handelt es sich bei den Terminationssequenzen typischerweise um Stopcodons und entsprechende regulatorische Sequenzen; sollen die übertragenen Nukleinsäuren nur transkribiert werden, handelt es sich in der Regel um poly(A)-Sequenzen.

Wie hier verwendet, betrifft der Begriff "Vektor" ein Nukleinsäuremolekül, das eine andere Nukleinsäure, an welche es gebunden ist, in eine Zelle transportieren kann. Ein Vektortyp ist ein "Plasmid", was für eine zirkuläre doppelsträngige DNA-Schleife darstellt, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff "Vektor" auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

In einem rekombinanten Expressionsvektor bedeutet "operativ daran gebunden", dass die Nukleotidsequenz von Interesse derart an die Regulationssequenz(en) gebunden ist, dass die Expression der Nukleotidsequenz möglich ist und die beiden Sequenzen derart aneinander gebunden sind, dass sie die vorhergesagte, der Sequenz zugeschriebene Funktion erfüllen.

Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder in Gruber und Crosby, in: Methods in Plant Molecular Biology and Biotechnology, CRC Press, Boca Raton, Florida, Hrsgb.: Glick und Thompson, Kapitel 7, 89-108. Regulationssequenzen umfassen solche, welche die konstitutive Expression einer Nukleotidsequenz in vielen Wirtszelltypen steuern, und solche, die die direkte Expression der Nukleotidsequenz nur in bestimmten Wirtszellen unter bestimmten Bedingungen steuern. Der Fachmann weiß, dass die Gestaltung des Expressionsvektors von Faktoren wie der Auswahl der zu transformierenden Wirtszelle, dem gewünschten Ausmaß der Expression des Proteins usw., abhängen kann.

Die verwendeten rekombinanten Expressionsvektoren zur Expression der Peroxidase können sowohl in prokaryotischen als auch in eukaryotischen Zellen aktiv sein. Dies ist vorteilhaft, da häufig Zwischenschritte der Vektorkonstruktion der Einfachheit halber in Mikroorganismen durchgeführt werden. Diese Klonierungsvektoren enthalten ein Replikationssignal für den entsprechenden Mikroorganismus und ein Markergen zur Selektion erfolgreich transformierter Bakterienzellen. Zur Expression in prokaryotischen Organismen geeignete Vektoren sind dem Fachmann bekannt, zu diesen gehören z.B. in *E. coli* pLG338, pACYC184, die pBR-Reihe, wie z.B. pBR322, die pUC-Reihe, wie z.B. pUC18 oder pUC19, die M113mp-Reihe, pKC30, pRep4, pHS1, pHS2, pPLc236, pMBL24, pLG200, pUR290, pIN-III113-B1, λgt11 oder pBdCl, in *Streptomyces* pIJ101, pIJ364, pIJ702 oder pIJ361, in *Bacillus* pUB 110, pC194 oder pBD214, in *Corynebacterium* pSA77 oder pAJ667.

Bei einer weiteren Ausführungsform stellt der Expressionsvektor einen Hefeexpressionsvektor oder einen Bacillovirus-Expressionsvektor dar.

Die oben genannten Vektoren bieten nur einen kleinen Überblick über mögliche geeignete Vektoren. Weitere Plasmide sind dem Fachmann bekannt und sind z.B. beschrieben in: Cloning Vectors (Hrsg. Pouwels, P.H. et al. Elsevier, Amsterdam, New York-Oxford, 1985). Weitere geeignete Expressionssysteme für prokaryotische und eukaryotische Zellen siehe in den Kapiteln 15 und 16 von Sambrook und Russell, vide supra.

Bei einer weiteren Ausführungsform des Verfahrens kann die Peroxidase in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992), Plant Mol. Biol. 20:1195-1197; und Bevan, M.W. (1984), Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Da die Pflanzengenexpression sehr oft nicht auf die Transkriptionsebene beschränkt ist, enthält eine Pflanzen-Expressionskassette vorzugsweise zusätzlich zu den oben beschriebenen Elemente andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al. (1987) Nucl. Acids Research 15:8693-8711).

Das zu exprimierende Gen muss wie oben beschrieben funktionsfähig mit einem geeigneten Promotor verbunden sein, der die Genexpression auf rechtzeitige, zell-oder gewebespezifische Weise steuert. Geeignete Promotoren wurden bereits oben beschrieben.

Andere bevorzugte Sequenzen für die Verwendung zur funktionsfähigen Verbindung in Genexpressionskassetten sind Targeting-Sequenzen, die zur Steuerung des Genproduktes in das entsprechende Zellkompartiment notwendig sind (siehe eine Übersicht in Kermode (1996) Crit. Rev. Plant Sci. 15 (4): 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Zum Einbringen der Peroxidase-Sequenzen in die Expressionsvektoren werden diese vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Bakterien, Hefen oder Pilzen gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakteriumvermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E. *coli* als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Binl9, pBI101, pBinAR, pGPTV und pCAMBIA. Eine Übersicht über binäre Vektoren und ihre Verwendung gibt Hellens et al. (2000) Trends in Plant Science 5, 446-451.

Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung werden das enzymatisch geschnittene und erforderlichenfalls gereinigte Amplifikat mit ähnlich präparierten Vektorfragmenten durch eine Ligase miteinander verknüpft. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere *E. coli* und *Agrobacterium tumefaciens*, in einem geeigneten Medium züchten und unter Selektionsbedingungen stabil propagieren. Anschließend werden die Zellen geerntet und lysiert und das Plasmid daraus gewonnen. Dies ermöglicht einen Transfer von heterologer DNA in Pflanzen oder Mikroorganismen.

Unter der vorteilhaften Verwendung von Klonierungsvektoren können die im erfindungsgemäßen Verfahren verwendeten Nukleinsäuren, die erfinderischen Nukleinsäuren und Nukleinsäurekonstrukte in Organismen wie Mikroorganismen oder bevorzugt Pflanzen eingebracht werden und zur Pflanzentransformation verwendet werden, wie diejenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus (1991) Annu. Rev. Plant Physiol. Plant Molec. Biol. 42: 205-225. Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen, bevorzugt von Pflanzen, verwenden.

Für die Einführung von DNA in eine pflanzliche Wirtszelle stehen eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten. Dabei können sowohl stabile als auch transiente Transformanten erzeugt werden.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig. Dem Fachmann sind die gängigen Selektionsmarker bekannt und es stellt für ihn kein Problem dar, einen geeigneten Marker auszuwählen. Gebräuchliche Seklektionsmarker sind solche, die den transformierten Pflanzenzellen Resistenz gegenüber einem Biozid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und dergleichen vermitteln. Der individuell gewählte Marker sollte die Selektion transformierter Zellen gegenüber Zellen, denen die eingeführte DNA fehlt, gestatten. Hierzu sind auch alternative Marker geeignet, wie nutritive Marker oder Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgene Pflanzen erwünscht sind, stehen dem Fachmann auch Strategien zur Verfügung, die eine nachträgliche Entfernung des Markergens erlauben, z. B. Cotransformation oder Sequenz-spezifische Rekombinasen. Ist die eingeführte DNA einmal im Genom der Pflanzenzelle integriert, so ist sie dort in der Regel stabil und bleibt auch in den Nachkommen der ursprünglich transformierten Zelle erhalten.

Werden für die Transformationen Agrobakterien verwendet, muss die einzuführende DNA, wie oben bereits ausgeführt, in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E. coli als auch in Agrobakterien replizieren. Sie enthalten ein Selektionsmarker-Gen und einen Linker oder Polylinker, welche von der rechten und linken T-DNA-Grenzregion eingerahmt werden. Sie können direkt in die Agrobakterien transformiert werden (Holsters et al. (1978), Molecular and General Genetics 163, 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzlich kann T-DNA vorhanden sein. Das derartig transformierte Agrobakterium wird zur Transformation von Pflanzenzellen verwendet.

Die Verwendung von T-DNA für die Transformation von Pflanzenzellen ist intensiv untersucht und ausreichend in EP 120 515 beschrieben worden

Für den Transfer der DNA in die Pflanzenzelle können Pflanzen-Explantate zweckmäßigerweise mit Agrobacterium tumefaciens oder Agrobacterium rhizogenes kultiviert werden. Aus dem infizierten Pflanzenmaterial (beispielsweise Blattstücke, Stengelsegmente, Wurzeln, aber auch Protoplasten oder Suspensions-kultivierte Pflanzenzellen) können dann in einem geeignetem Medium, welches Antibiotika oder Biozide zur Selektion transformierter Zellen enthalten kann, wieder ganze Pflanzen regeneriert werden. Die Regeneration der Pflanzen erfolgt nach üblichen Regenerationsmethoden unter Verwendung bekannter Nährmedien. Die so erhaltenen Pflanzen bzw. Pflanzenzellen können dann auf Anwesenheit der eingeführten DNA mit etablierten Methoden wie z.B. Southern Blot oder PCR untersucht werden.

Andere Möglichkeiten der Einführung fremder DNA unter Verwendung des biolistischen Verfahrens oder durch Protoplasten-Transformation sind dem Fachmann bekannt (vgl. L. Willmitzer (1993) Transgenic Plants in: Biotechnology, A Multi-Volume Comprehensive Treatise (Herausgeber: H.J. Rehm et al.), Band 2, 627-659, VCH Weinheim, Deutschland).

Inzwischen ist nicht nur die Transformation dikotyler Pflanzen bzw. derer Zellen über Ti-Plasmid-Vektorsysteme mit Hilfe von Agrobacterium tumefaciens gut etabliert, sondern auch die monokotyler Pflanzen bzw. derer Zellen (siehe u.a. Chan et al. (1993), Plant Mol. Biol. 22, 491-506).

Alternative Systeme zur Transformation von monokotylen Pflanzen bzw. deren Zellen sind die Transformation mittels des biolistischen Ansatzes (Wan und Lemaux (1994) Plant Physiol. 104, 37-48; Vasil et al. (1993) Bio/Technology 11, 1553-1558; Ritala et al. (1994) Plant Mol. Biol. 24, 317-325; Spencer et al. (1990), Theor. Appl. Genet. 79, 625-631), die Protoplasten-Transformation, die Elektroporation von partiell permeabilisierten Zellen sowie die Einbringung von DNA mittels Glasfasern.

Die transformierten Zellen wachsen innerhalb der Pflanze in üblicher Weise (siehe auch McCormick et al. (1986), Plant Cell Reports 5, 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen besitzen die entsprechenden phänotypischen Eigenschaften.

Es sollten zwei oder mehrere Generationen angezogen werden, um sicherzustellen, dass das phänotypische Merkmal stabil beibehalten und vererbt wird. Auch sollten Samen geerntet werden, um sicherzustellen, dass der entsprechende Phänotyp oder andere Eigenarten erhalten geblieben sind.

Ebenso können nach üblichen Methoden transgene Linien identifiziert werden, die für die neuen Nukleinsäuremoleküle homozygot sind und ihr phänotypisches Verhalten hinsichtlich einer vorhandenen bzw. nicht vorhandenen Pathogen-Responsivität untersucht und mit dem von hemizygoten Linien verglichen werden. Selbstverständlich können die Pflanzenzellen, die die erfindungsgemäßen Nukleinsäuremoleküle enthalten, auch in Form einer Zellkultur (einschließlich Protoplasten, Kalli, Suspensionskulturen und dergleichen) weiterkultiviert werden.

Das erfindungsgemäße Verfahren kann vorteilhaft mit weiteren Verfahren, die eine Pathogenresistenz (beispielsweise gegen Insekten, Pilze, Bakterien, Nematoden, usw.), Stressresistenz oder eine andere Verbesserung der pflanzlichen Eigenschaften bewirken, kombiniert werden. Beispiele sind u.a. genannt in Dunwell JM (2000) J Exp Bot. 51: 487-496.

Der Begriff transgene Pflanze umfasst sowohl die Pflanze in ihrer Gesamtheit, als auch alle Pflanzenteile, in denen die Expression von pflanzlichen Peroxidaseproteinen erhöht ist. Dazu gehören alle Pflanzenteile und Pflanzenorgane wie Blatt, Stiel, Samen, Wurzel, Knollen, Antheren, Fasern, Wurzelhaare, Stengel, Embryos, Kalli, Kotelydonen, Petiolen, Erntematerial, pflanzliches Gewebe, reproduktives Gewebe, Zellkulturen, die sich von der transgenen Pflanze ableiten und/oder dazu verwendet werden können, die transgene Pflanze hervorzubringen.

Je nach dem verwendeten Vektorsystem können erfindungsgemäß auch transgene Pflanzen hergestellt werden, bei denen die zu übertragenden Nukleinsäuren in der Pflanzenzelle bzw, der Pflanze als selbstständig replizierendes System enthalten sind. Die zur Übertragung der Pflanzen verwendeten Vektoren müssen dann entsprechend über DNA-Sequenzen verfügen, die die Replikation von zur Übertragung verwendeten Plasmiden innerhalb der Zelle ermöglichen.

Die spezifische Expression des Peroxidase-Proteins in den erfindungsgemäßen Pflanzen bzw. in den erfindungsgemäßen Pflanzenzellen kann mit Hilfe herkömmlicher molekularbiologischer und biochemischer Methoden nachgewiesen und verfolgt werden. Dem Fachmann sind diese Techniken bekannt und er ist problemlos in der Lage, eine geeignete Nachweismethode zu wählen, beispielsweise eine Northern-Blot-Analyse zum Nachweis Peroxidase-spezifischer RNA bzw. zur Bestimmung der Höhe der Akkumulation von Peroxidase-spezifischer RNA oder eine Southern-Blot- bzw. PCR-Analyse zum Nachweis von für die Peroxidase kodierenden DNA-Sequenzen. Die dazu verwendeten Sonden- bzw. Primersequenzen können zu der in SEQ ID No. 1 angegebenen Sequenz entweder identisch sein oder einige wenige Abweichungen von dieser Sequenz aufweisen.

Die oben beschriebenen Techniken können selbstverständlich auch dazu verwendet werden, weitere Pflanzen zu identifizieren, die aufgrund des Vorhandenseins der im Rahmen der vorliegenden Erfindung identifizierten Peroxidase eine Nicht-Wirts-Resistenz aufweisen.

Bei den für das erfindungsgemäße Verfahren verwendeten Pflanzen kann es sich im Prinzip um jede beliebige Pflanze handeln, die resistent gegen Pathogenbefall gemacht werden soll. Vorzugsweise ist es eine monokotyle oder dikotyle Nutz-, Nahrungs- oder Futterpflanze.

Beispiele für monokotyle Pflanzen sind Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören.

Dikotyle Nutzpflanzen umfassen unter anderem Baumwolle, Leguminosen wie Hülsenfrüchte und insbesondere Alfalfa, Sojabohne, Raps. Canola, Tomate, Zuckerrübe, Kartoffel, Sonnenblume, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitalis umfassen. Besonders bevorzugt sind die Getreide Weizen, Roggen, Hafer, Gerste, Reis, Mais und Hirse, sowie die dikotylen Pflanzen Zuckerrübe, Raps, Soja, Tomate, Kartoffel und Tabak. Weitere Nutzpflanzen können dem US-Patent Nr. 6,137,030 entnommen werden.

Bevorzugte Pflanzen sind Tagetes, Sonnenblume, Arabidopsis, Tabak, Roter Pfeffer, Soja, Tomate, Aubergine, Paprika, Möhre, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaeen wie beispielsweise Raps oder Canola, Zuckerrübe, Zuckerrohr, Nuss- und Weinspezies oder Holzgewächse wie beispielsweise Espe oder Eibe.

Die erfindungsgemäßen transgenen Pflanzen und die von ihnen abgeleiteten Zellen, Zellkulturen, Teile und transgene Vermehrungsmaterialien Können zur erstellung von Nahrungs- und Futtermitteln, Pharmazeutika oder Feinchemikalien verwendet werden.

Die Identifizierung der Peroxidase aus Gerste als Gen, das eine Nicht-Wirts-Resistenz der Gerste gegenüber Isolaten von *Blumeria graminis f. sp. triticum* vermittelt, und deren Anwendung zur Vermittlung der Pathogenresistenz in transgenen Pflanzen bzw. Pflanzenzellen wird nun im Folgenden dargestellt. Die nachfolgenden Beispiele sollen nicht als beschränkend aufgefasst werden.

<u>Beispiele</u>

Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose- und Nylon-Membranen, Verknüpfen von DNA-Fragmenten, Transformation von *E. coli*-Zellen, Anzucht von Bakterien und die Sequenzanalyse rekombinanter DNA wurden wie bei Sambrook et al. (2001), vide supra, beschrieben durchgeführt.

Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA 74, 5463-5467).

Beispiel 3: Identifizierung von HvBgt1 aus Gerste durch Oligonucleotide Fingerprinting

Das Peroxidase-Gen HvBgt1 aus Gerste wurde durch Oligonucleotide Fingerprinting (Radelof et al. (1998) Nucleic Acids Res. 26(23):5358-64) als Bgt-induziertes Gen identifiziert. Diese Methode erlaubt die Identifizierung von Expressionsmustern auch sehr niedrig exprimierter Gene.

HvBgt1 wurde in einer Bibliothek identifiziert, die aus mRNA aus Gerstepflanzen, die mit Weizenmehltau (Bgt; Erntezeitpunkte des Blattgewebes: 6, 24, 48 und 72 h.p.i.) infiziert worden waren, generiert wurde. Unter Verwendung der von der BASF Corporation entwickelten Software HyStac wurde die Abundanz der einzelnen Cluster in dieser Bibliothek mit derjenigen zweier weiterer Gerste-Bibliotheken (Gerste inokuliert mit Gerstenmehltau (Bgh) sowie Gerste, nicht inokuliert), die als Referenz dienten, verglichen. Für HvBgt1 ergab sich in dieser Analyse die Vorhersage einer ca. 10fach erhöhten Abundanz in der Gerste + Bgt-Bibliothek.

Beispiel 4: Kultur der Gerstelinien und Infektion mit Mehltau

Für die Experimente wurde die Gerste-Wildtyp-Linie Ingrid *MLO*Bc verwendet. Die Samen wurden von Dr. Patrick Schweizer, IPK Gatersleben, zur Verfügung gestellt.

Die Samen wurden nach dem Auslegen auf Erde zur Stratifizierung für 24 h bei 4˚C inkubiert. Danach wurden die Pflanzen bei kontrollierten Wachstumsbedingungen ins Gewächshaus (Agrarzentrum Limburgerhof, BASF AG) gestellt. Die Temperatur betrug im Mittel 23˚C, die Luftfeuchtigkeit zwischen 40 und 70%. Der Tag/Nacht-Rhythmus betrug 10h und 14 h. Die Pflanzen wurden 7 Tage nach der Aussaat mit *Bgh* bzw. *Bgt* inokuliert. Das für die Versuche eingesetzte Isolat FA6h des Erregers *Blumeria graminis f. sp. hordei* wurde von der Forschungsstation der ETH Zürich in Heckenholz zur Verfügung gestellt. Der Erreger *Blumeria graminis f. sp. tritici* ist ein Feldisolat, welches im Agrarzentrum der BASF AG auf der Weizen-Sorte Kanzler kultiviert wird. Zur Infektion wurden stark mit *Bgh* infizierte Gerste- bzw. mit *Bgt* infizierte Weizen-Pflanzen über die zu infizierenden Gerste- bzw. *Arabidopsis*-Pflanzen gehalten und der Mehltau abgeschüttelt, um die Konidien auf die Pflanze zu übertragen.

Beispiel 5: Isolation der Gesamt-RNA aus infizierten Gerstepflanzen

Über einen Zeitraum von vier Tagen wurden jeweils nach 24 Stunden infiziertes Gerstematerial und nicht infizierte Kontrollen geerntet, in Alufolie gepackt und in Trockeneis tiefgefroren. Die Aufbewahrung des Blattmaterials erfolgte bei -80˚C. Nach dem Zerkleinern des Blattmaterials wurde die Gesamt-RNA mit Hilfe des RNeasy Plant Maxi Kit® (Qiagen, Hilden, Deutschland) nach Herstellerangaben isoliert. Die Elution der gereinigten RNA erfolgte mit 2 x 0,6 ml RNase-freiem Wasser. Die Konzentration wurde mit dem EPPENDORF BioPhotometer 6131 bestimmt, anschließend wurde die RNA mit 2 Volumenteilen 98% Ethanol und 1/10 Volumenteil Na-Acetat (3 M, pH 5,2) gefällt und auf eine Konzentration von ca. 2 µg/µl eingestellt.

Beispiel 6: Ermittlung der Peroxidase-Expression durch quantitative PCR-Analysen

Für die quantitative PCR wurden die aus dem Blattmaterial isolierten RNA-Proben eingesetzt. Zunächst wurde eventuell noch in den RNA-Proben vorhandene DNA verdaut. Der Verdau wurde mit DNA-free™ der Firma AMBION (Huntingdon, USA) wie folgt angesetzt:

| | |
|---|---|
| RNA | 50 µl |
| 10x DNase I Puffer | 6 µl |
| DNase I (2 U/µl) | 2 µl |
| $H_2O$ ad 60 µl | 2 µl |

Der Ansatz wurde für 30 min bei 37˚C inkubiert. Anschließend wurden 9 µl DNase Inactivation Reagent zugegeben und der Ansatz gut gemischt. Nach einer weiteren Inkubationszeit von 2 min bei Raumtemperatur wurde die Lösung bei 10000 g für 1 min zentrifugiert, um das DNase Inactivation Reagent zu pelletieren. Die RNA wurde in ein neues Gefäß überführt und bei -20˚C aufbewahrt.

Nach dem DNase-Verdau wurde die RNA revers in cDNA transkribiert. Der Ansatz erfolgte mit den Taq Man Reverse Transcription Reagents der Firma APPLIED BIOSYSTEMS (Applera Deutschland GmbH, Darmstadt):

| | |
|---|---|
| RNA | 6 µl |
| 25 mM $MgCl_2$ | 4,4 µl |
| dNTP-Mix (10 mM) | 4 µl |
| 50 µM Random Hexamer | 1 µl |
| 11x RT Puffer | 2 µl |
| RNase Inhibitor | 0,4 µl |
| Multiscribe RT (50 U/µl) | 1,5 µl |
| $H_2O$ Nuklease-frei | 0,7 µl |

Der Ansatz wurde für 10 min bei 25˚C inkubiert, anschließend erfolgte eine Inkubation bei 37˚C für 60 min. Schließlich wurde der Ansatz für 5 min bei 95˚C hitzeinaktiviert.

Die transkribierte DNA wurde mit 20 µl $H_2O$ verdünnt und davon jeweils 2,5 µl für die quantitative PCR eingesetzt. Als interner Standard wurde die 18S rRNA mitbestimmt. Von allen Proben wurde eine Dreifachbestimmung durchgeführt. Die Ansätze wurden in eine 96 Napf-Platte pipettiert. Zunächst wurde die cDNA einzeln vorgelegt, dann wurde der SYBR Green® Master Mix mit den Primern und der entsprechenden Menge Wasser einzeln dazu pipettiert und der Ansatz gemischt.

| | |
|---|---|
| cDNA | 2,5 µl |
| 2x SYBR Green® Master Mix | 12,5 µl |
| forward Primer, Gip 162, 200 nM | 0,09 µl |
| reverse Primer, Gip 163, 200 nM | 0,14 µl |
| $H_2O$ Nuklease-frei ad 25 µl | 9,77 µl |

| | |
|---|---|
| Gip 162: | CGAGGCCCTTGTGACATACAT |
| Gip 163: | ACTTAGGTGTCGTTACTTGGACCAT |

Als Referenzprobe diente der jeweilige Kontrollwert vor der Infektion (0 h). Die Bestimmung der Transkriptmenge erfolgte mit Proben, die 24 h, 48 h und 72 h nach der Infektion mit *Bgh* genommen wurden.

Ansatz für die 18S rRNA:

| | |
|---|---|
| cDNA | 2,5 μl |
| 2x SYBR Green® Master Mix | 12,5 μl |
| forward Primer, Gip 63, 200 nM | 0,12 μl |
| reverse Primer, Gip 64, 200 nM | 0,13 μl |
| $H_2O$ Nuklease-frei | 9,75 μl |

| | |
|---|---|
| Gip 63: | CGTCCCTGCCCTTTGTACAC |
| Gip 64: | AACACTTCACCGGACCATTCA |

Die Platte wurde bei Raumtemperatur und 2500 rpm für 1 min zentrifugiert, danach wurden die Proben direkt im Gerät ABI PRISM 7000 der Firma APPLIED BIOSYSTEMS (Applera Deutschland GmbH, Darmstadt) vermessen. Die Auswertung erfolgte mit Hilfe des Programms ABI PRISM 7000 SDS der Firma APPLIED BIOSYSTEMS.

Die mit der quantitativen PCR ermittelten Expressionsdaten sind in Tabelle 5 und Abb. 1 dargestellt. Die Messung wurde zweimal durchgeführt und eine dreifache Bestimmung der einzelnen Messwerte vorgenommen. Gezeigt sind die jeweils gemittelten Werte und die dazu gehörige Standardabweichung.

Tabelle 5: Darstellung des zeitlichen Verlaufs der Expression von *HvBgt1* bei der Nicht-Wirts- (Gerste und *Bgt*) und der Wirts-Interaktion (Gerste mit *Bgh*)

| Proben-nummer | Pflanzen-material | Gen-expression | Standard-abweichung | Kalibrator |
|---|---|---|---|---|
| 1 | Ingrid Kont. 0h | 1,0 | 0,1 | Ingrid 0 h Kontrolle |
| 2 | Ingrid Kont. 24 h | 6,1 | 3,5 | |
| 3 | Ingrid Kont. 48 h | 28,2 | 4,9 | |
| 4 | Ingrid Kont. 72 h | 25,1 | 3,4 | |
| 5 | Ingrid + *Bgt* 0 hpi | 1,0 | 0,3 | Ingrid + *Bgt* 0 hpi |
| 6 | Ingrid + *Bgt* 24 hpi | 107,1 | 18,3 | |
| 7 | Ingrid + *Bgt* 48 hpi | 25,0 | 12,9 | |
| 8 | Ingrid + *Bgt* 72 hpi | 10,6 | 5,0 | |
| 9 | Ingrid + *Bgh* 0 hpi | 1,0 | 0,1 | Ingrid + *Bgh* 0 hpi |
| 10 | Ingrid + *Bgh* 24 hpi | 1,5 | 0,6 | |
| 11 | Ingrid + *Bgh* 48 hpi | 0,5 | 0,1 | |
| 12 | Ingrid + *Bgh* 72 hpi | 3,1 | 1,2 | |

Als Vergleichswert bzw. Kalibrator diente für jede Interaktion der 0 h-Wert der Messung.

Die Ergebnisse zeigen einen deutlichen Unterschied in der *HvBgt1*-Expression zwischen den drei Systemen Kontrolle, Nicht-Wirts-Interaktion und Wirts-Interaktion. In der Kontrolle ist bis zum Zeitpunkt 48 h ein deutlicher Anstieg der Transkriptmenge um das etwa 28-fache zu verzeichnen, der sich dann bis 72 h nach dem Beginn der Messung fast konstant hält. Bei der Nicht-Wirts-Interaktion (Gerste-*Bgt*) ist dagegen schon nach 24 h ein Anstieg um fast das 110-fache zu erkennen, der dann nach 72 h wieder langsam auf die 10-fache Menge reduziert wird. Dagegen ist in der Wirts-Interaktion (Gerste-*Bgh*) über den gesamten Verlauf nur eine maximal dreifache Erhöhung der Transkriptmenge zu erkennen.

Beispiel 7: Isolierung der Volllängensequenz von HvBgt1 aus Gerste durch RACE-PCR

Für die RACE-PCR wurde die aus dem infizierten Blattmaterial isolierte RNA eingesetzt. Die RACE-cDNA-Bank wurde mit dem GeneRacer™-Kit der Firma Invitrogen (Karlsruhe, Deutschland) nach Herstellerangaben hergestellt. Als Gen-Spezifische Primer (GSP) wurden als 5'-RACE-Primer der Primer M207 und als nested primer der Primer M208 verwendet.

| | |
|---|---|
| M207: | GCTTATTCAGCAGCCAACAAAGTAAC |
| M208: | CTGTTTCACAAGTTTATGGTCCAAGTAA |

Ansatz der Proben-PCR:

| | |
|---|---|
| 10x Polymerase-Puffer | 5 µl |
| dNTP-Mix (10 mM), INVITROGEN | 1,5 µl |
| Template (Hv-RACE-Bank) | 1 µl |

| | |
|---|---|
| GSP, M 207 (10 pmol) | 4 µl |
| GeneRacer X'Primer (10 pmol) | 4,5 µl |
| Polymerase (5 U/µl) | 1 µl |
| $H_2O$ | 33 µl |

PCR-Programm

| | | | |
|---|---|---|---|
| Denaturierung | 95°C | 2 min | |
| Denaturierung | 95°C | 30 sek | 25x |
| Anlagerung (Annealing) | 65°C | 30 sek | |
| Polymerisierung | 68°C | 2 min | |
| abschließende Polymerisierung | 68°C | 10 min | |

Ansatz für die Proben nested PCR:

| | |
|---|---|
| 10x Polymerase-Puffer | 5 µl |
| dNTP-Mix (10 mM), INVITROGEN | 1,5 µl |
| Template (PCR-Ansatz) | 5 µl |

(fortgesetzt)

| | |
|---|---|
| GSP, M 207 (10 pmol) | 4 μl |
| GeneRacer X' Nested Primer, M 208 (10 pmol) | 4,5 μl |
| Polymerase (5 U/μl) | 1 μl |
| $H_2O$ | 33 μl |

PCR-Programm

| | | | |
|---|---|---|---|
| Denaturierung | 95°C | 2 min | |
| Denaturierung | 95°C | 30 sek | 25x |
| Anlagerung (Annealing) | 65°C | 30 sek | |
| Polymerisierung | 68°C | 2 min | |
| abschließende Polymerisierung | 68°C | 10 min | |

Die Volllängensequenz des Gens *HvBgt1* wurde in mehreren Teilschritten amplifiziert, anschließend sequenziert und dann *in silico* zusammengesetzt. Nachdem durch die 3'RACE ein Sequenzstück mit Poly-A-Extension und mit der 5'-RACE ein weiteres Sequenzstück amplifiziert wurde, konnten über ein *Contig* die neuen Sequenzen mit der Ursprungssequenz zusammengefügt werden. Die Gesamtsequenz betrug ca. 1000 bp. Mit Hilfe des Computerprogramms ContigExpress (INFORMAX, Maryland, USA) wurden alle ORFs, die in der Sequenz möglich sind, angezeigt. Es konnte ein ORF gefunden werden, der sich über die Länge von 945 bp erstreckt. Um zu überprüfen, ob das Gen vollständig ist, wurde nach einem weiteren Stop-Codon vor dem Start gesucht. Das Stop-Codon konnte nach 3 Triplets in 5'-Richtung gefunden werden.

Um das Gen als Volllängen-Klon zu erhalten, wurden Primer ausgewählt, die die Amplifikation des gesamten Gens erlauben (end-to-end-PCR). Das PCR-Fragment wurde dann aus dem Agarose-Gel aufgereinigt und in den pCR®4-TOPO®-Vektor kloniert. Die Volllängennukleotidsequenz von HvBgt1 ist in SEQ ID No. 1 und die Aminosäuresequenz ist in SEQ ID No. 2 dargestellt.

Beispiel 8: Identifizierung und Klonierung der zu *HvBgt1* homologen Gene in *Arabidopsis thaliana*

Nachdem die Volllängensequenz der Gersten-Peroxidase (*HvBgt1*) erhalten worden war, wurde mit dieser Sequenz eine BLAST-Suche gegen das *Arabidopsis*-Genom durchgeführt. Dabei wurden zwei homologe Gene, *AtBgt1-1* und *AtBgt1-2*, identifiziert. Diese beiden Gene wurden durch PCR mit geeigneten Primern aus *A. thaliana*-cDNA amplifiziert und in den Pflanzenexpressionsvektor pCambia mit konstitutivem Promotor kloniert, um eine Überexpression in *A. thaliana* zu ermöglichen.

Zunächst wurde die Gesamt-RNA aus einer Mischung von nichtinfizierten Arabidopsis-Pflanzen und Arabidopsis-Pflanzen, die mit Alternaria alternata und Alternaria brassicola infiziert worden waren, unter Verwendung des Superscript First Strand Synthesis System for RT-PCR® (INVITROGEN, Karlsruhe) in cDNA umgeschrieben.

Zur Amplifikation der Volllängensequenz der Gene aus der cDNA durch PCR mit Platinum Pfx-Polymerase (INVITROGEN, Karlsruhe, Deutschland) wurden anhand der *AtBgt1-1*-bzw. *AtBgt1-2*-Sequenzen Primer abgeleitet. Die zu amplifizierende Fragmentgröße für *AtBgt1-2* sollte 1015 bp sein. Die Amplifikation der *AtBgt1-2*-Sequenz erfolgte mit den beiden Primern Fra335 und Fra336 nach dem Protokoll:

| | |
|---|---|
| 10x Polymerase-Puffer, STRATAGENE (La Jolla, USA) | 5 μl |

(fortgesetzt)

| | |
|---|---|
| dNTP-Mix (10 mM), INVITROGEN (Karlsruhe, Deutschland) | 1 µl |
| cDNA | 1 µl |
| Fra335 (20 pmol) | 1 µl |
| Fra336 (20 pmol) | 1 µl |
| Taq-Polymerase (5 U/µl) | 1 µl |
| $H_2O$ | 40 µl |

Fra 335: AGAAGTATGTTAAAGGTGGTGTTGTTG
Fra 336: TGACGAAGAGGTGATTATTGAGC

PCR-Programm

| | | | |
|---|---|---|---|
| Denaturierung | 95°C | 5 min | |
| Denaturierung | 95°C | 30 sek | 35x |
| Anlagerung (Annealing) | 55,9°C | 30 sek | |
| Polymerisierung | 72°C | 2 min | |
| abschließende Polymerisierung | 72°C | 10 min | |

Nach der Aufreinigung der PCR-Fragmente aus dem Gel wurden die Sequenzen in den pCR®Blunt-II-TOPO® Vektor (INVITROGEN, Karlsruhe) zwischenkloniert und in TOP10®-*E.coli*-Zellen transformiert. Über eine Blau-Weiß-Selektion wurden Kolonien ausgewählt, die das Konstrukt enthalten. Von diesen Kolonien wurden Minikulturen angeimpft, um Plasmid-DNA zu isolieren. Für jedes Fragment wurden drei Klone sequenziert (DNA-Labor, BASF AG, Ludwigshafen). Die Klone zeigten 100% übereinstimmende Sequenzen und wurden mit *Eco*RI aus dem TOPO®-Vektor ausgeschnitten und über Gelelektrophorese aufgetrennt. Die Fragmente wurden aus dem Gel aufgereinigt und in den Vektor pCambia, der mit *Eco*RI geschnitten worden war, ligiert.

Beispiel 9: Transiente Transformation von Gerstezellen, Überexpression und Evaluation der Pilzpathogenentwicklung

Blattsegmente von Gerste cv Pallas wurden mit der *HvBgt1*-DNA zusammen mit einem GFP-Expressionsvektor transformiert. Anschließend wurden die Blätter mit Bgh inokuliert und das Ergebnis nach 48 h mittels Licht- und Fluoreszenzmikroskopie analysiert. Die Penetration von GFP-exprimierenden Zellen wurde mittels Detektion von Haustorien in lebenden Zellen und durch Bewertung der Pilzentwicklung in eben diesen Zellen beurteilt. In allen sechs Experimenten führte die Bombardierung von Gerste cv Pallas mit *HvBgt1* zu einer verminderten Anzahl von erfolgreich durch Bgh penetrierten Zellen im Vergleich zu Zellen, die mit einer fremden Kontroll-DNA (humaner Thyroidhormonrezeptor, TR) bombardiert worden waren.

Zur transienten Transformation der Gerstezellen wurde ein Verfahren eingesetzt, das bereits für die biolistische Einführung von DNA in epidermale Zellen von Gerstenblättern beschrieben wurde (Schweizer P et al. (1999) Mol Plant Microbe Interact 12:647-54). Wolframpartikel mit einem Durchmesser von 1,1 µm (Partikeldichte 25 mg/ml) wurden mit *HvBgt1*-DNA zusammen mit Plasmid-DNA des Vektors *pGFP* (GFP unter Kontrolle des pUBI Promotors) als Transformationsmarker beschichtet. Dazu wurden pro Schuss die nachfolgenden Mengen an DNA bzw. Reporterplasmid zur Beschichtung verwendet: 1 µg pGFP und 2 µg DNA.

Für die Microcarrier-Präparation wurden 55 mg Wolframpartikel (M 17, Durchmesser 1,1 µm; Bio-Rad, München) zweimal mit 1 ml autoklaviertem destilliertem Wasser und einmal mit 1 mL absolutem Ethanol gewaschen, getrocknet und in 1 ml 50 % Glycerin aufgenommen (ca. 50 mg/ml Stammlösung). Die Lösung wurde mit 50 % Glycerin auf 25 mg/ml verdünnt, vor Gebrauch gut gemischt und im Ultraschallbad suspendiert. Zur Microcarrier-Beschichtung wurden pro Schuss 1 µg Plasmid, 2 µg *HvBgt1*-DNA (1 µL), 12,5 µl Wolframpartikel-Suspension (25 mg/ml), 12,5 µl 1 M $Ca(NO_3)_2$-Lösung (pH 10) tropfenweise unter ständigem Mischen zusammengegeben, 10 min bei RT stehengelassen, kurz zentrifugiert und 20 µl vom Überstand abgenommen. Der Rest mit den Wolframpartikel wurde im Ultraschallbad resuspendiert und ins Experiment eingesetzt.

Zur Transformation wurden ca. 4 cm lange Segmente von Gerstenprimärblättem verwendet. Die Gewebe wurden auf 0,5 % Phytagar (GibcoBRL™ Life Technologies™,Karlsruhe) mit 20 µg/ml Benzimidazol in Petrischalen (6,5 cm Durchmesser) gelegt und direkt vor dem Partikelbeschuss an den Rändern mit einer Schablone mit einer rechteckigen Aussparung von 2,2 cm x 2,3 cm abgedeckt. Die Schalen wurden nacheinander auf den Boden der Vakuumkammer (Schweizer P et al. (1999) Mol Plant Microbe Interact 12:647-54) gestellt, über dem ein Nylonnetz (Maschenweite 0,2 mm, Millipore, Eschborn) als Diffusor auf einer Lochplatte eingeschoben ist (5 cm über dem Boden, 11 cm unterhalb des Macrocarriers, s.u.), um Partikelklumpen zu zerstreuen und den Partikelstrom abzubremsen. Der oben an der Kammer angebrachte Macrocarrier (Plastik-Sterilfilterhalter, 13 mm, Gelman Sciences, Swinney, UK) wurde je Schuss mit 5,8 µL DNA-beschichteten Wolframpartikeln (Microcarrier, s.u.) beladen. Mit einer Membranvakuumpumpe (Vacuubrand, Wertheim) wurde der Druck in der Kammer um 0,9 bar reduziert und die Wolframpartikel mit 9 bar Heliumgasdruck auf die Oberfläche des Pflanzengewebes geschossen. Sofort danach wurde die Kammer belüftet. Zur Markierung transformierter Zellen wurden die Blätter mit dem Plasmid pGFP (Vektor auf pUC18-Basis, CaMV 35S-Promoter/Terminator-Kassette mit insertiertem GFP-Gen; Schweizer P et al. (1999) Mol Plant Microbe Interact 12: 647-54; zur Verfügung gestellt von Dr. P. Schweizer, Institut für Pflanzengenetik (IPK), Gatersleben, Deutschland) beschossen. Vor dem Schießen eines anderen Plasmids wurde der Macrocarrier jeweils gründlich mit Wasser gereinigt. Nach vierstündiger Inkubation nach dem Beschuss bei leicht geöffneten Petrischalen, RT und Tageslicht wurden die Blätter mit 100 Konidien/$mm^2$ des Echten Gerstenmehltaupilzes (*Blumeria graminis f.sp. hordei*, Rasse A6) inokuliert und für weitere 36 bis 48 h unter gleichen Bedingungen inkubiert, bevor die Auswertung im Hinblick auf Infektionsanzeichen erfolgte.

Das Ergebnis (z.B. die Penetrationseffizienz, definiert als prozentualer Anteil angegriffener Zellen, die ein reifes Haustorium und eine Sekundärhyphae ("secondary elongating hyphae") zeigen), wurde mittels Fluoreszenz- und Lichtmikroskopie bestimmt. Eine Inokulation mit 100 Konidien/$mm^2$ ergibt eine Angriffsfrequenz von ca. 50 % der transformierten Zellen. Für jedes einzelne Experiment wurde eine minimale Anzahl von 100 Interaktionsstellen ausgewertet. Transformierte (GFP-exprimierende) Zellen wurden unter Anregung mit blauem Licht identifiziert. Es konnten drei verschiedene Kategorien von transformierten Zellen unterschieden werden:

1. Penetrierte Zellen, die ein leicht erkennbares Haustorium beinhalten. Eine Zelle mit mehr als einem Haustorium wurde als eine Zelle gewertet.

2. Zellen, die durch ein Pilz-Appressorium zwar angegriffen wurden, aber kein Haustorium beinhalten. Eine Zelle, die mehrfach von Bgh angegriffen wurde, aber kein Haustorium enthielt, wurde als eine Zelle gewertet.

3. Zellen, die nicht durch Bgh angegriffen sind.

Stomatazellen und Stomatanebenzellen wurden von der Bewertung ausgeschlossen. Oberflächenstrukturen von Bgh wurden mittels Lichtmikroskopie oder Fluoreszenzfärbung des Pilzes mit 0,1 % Calcofluor (w/v in Wasser) für 30 sec analysiert. Die Entwicklung des Pilzes kann leicht durch Fluoreszenzmikroskopie nach Anfärbung mit Calcofluor evaluiert werden. In HvBGt1-DNA transformierten Zellen entwickelt der Pilz zwar einen primären und einen appressorialen Keimschlauch ("Germ-Tube"), aber kein Haustorium. Haustoriumausbildung ist eine Vorbedingung für die Bildung einer Sekundärhyphae.

Die relative Penetrationseffizienz (RPE) errechnet sich als Quotient aus der Penetrationseffizienz bei Zellen, die mit *HvBgt1* transformiert worden waren und der Penetrationseffizienz bei Zellen, die mit Kontroll-DNA transformiert worden waren. Die prozentuale RPE (%-RPE) errechnet sich aus der RPE minus 1 und multipliziert mit 100.

$$RPE = \frac{[\text{PE bei } \mathit{HvBgt1}\text{-DNA transformierten Zellen}]}{[\text{PE bei Kontroll-DNA transformierten Zellen}]}$$

$$\%\text{-RPE} = \quad 100 * (\text{RPE-1})$$

Der %-RPE-Wert (Abweichung von der durchschnittlichen Penetrationseffizienz der Kontrolle) dient der Bestimmung der Suszeptibilität von Zellen, die mit *HvBgt1*-DNA transfiziert sind.

Bei fünf unabhängigen Versuchen wurde kein Unterschied zwischen der Transfektion mit der Kontroll-DNA und Wasser bezüglich der Penetrationseffizienz von *Bgh* beobachtet.

Um einen Einfluss der *HvBgt1*-DNA auf die Transformationsrate oder Überlebensrate der angegriffenen Zellen auszuschließen, wurde die Anzahl der GFP-exprimierenden Zellen zwischen Kontroll- und Hv*Bgt1*-DNA-Experimenten verglichen. Die *HvBgt1*-Überexpression hat interessanterweise keinen Einfluss auf die Gesamtanzahl oder die Anzahl der angegriffenen GFP-exprimierenden Zellen.

Beispiel 10: Transformation von *Arabidopsis thaliana* mit *HvBgt1* und Analyse der Pilzresistenz

Wildtyp *A.thaliana* Pflanzen (Columbia) wurden mit dem Agrobacterium tumefaciens-Stamm (EHA105) auf Grundlage einer modifizierten Methode (Steve Clough und Andrew Bent (1998) Plant J 16(6):735- 743) der Vacuum Infiltrationsmethode nach Bechtold et al.(Bechtold N et al. (1993) CR Acad Sci Paris, Life Sciences 316:1194- 1199) transformiert. Dazu wurde ein binärer Expressionsvektor, der für die A. tumefaciens-Transformation geeignet ist, wie z.B. pCambia, verwendet.

Samen der Agrobacterium-transformierten Primärtransformanten wurden auf Grundlage der Kanamycin-Resistenz selektioniert. Antibiotika-resistente Keimlinge wurden in Erde gepflanzt und als vollentwickelte Pflanzen zur biochemischen Analyse verwendet.

Zur Analyse der Resistenz der transgenen *Arabidopsis*-Pflanzen gegenüber pathogenen Pilzen wurden Inokulationen mit den biotrophen Pilzen *Peronospora parasitica* und *Erysiphe cichoracearum* vorgenommen.

a) Peronospora parasitica

5 bis 8 Wochen alte Pflanzen wurden mit einer Konidiensporensuspension (ca. $10^6$ Sporen / ml) besprüht. Die inokulierten Pflanzen wurden über Nacht in einem Kühlschrank bei ca. 16˚C mit einer Plastiktüte überdeckt dunkel und feucht gehalten. Nach einem Tag wurde die Plastiktüte etwas geöffnet und später vollständig entfernt. Sechs Tage nach Inokulation werden die Pflanzen wieder über Nacht mit der Plastiktüte zugedeckt, wodurch die Sporulation induziert wurde. Am folgenden Tag wurden die Blätter auf das Auftreten von Konidiophoren untersucht. Das interzelluläre Wachstum des Pilzes führte in den nächsten Tagen zur Induktion von schwachen Chlorosen bis hin zu starken Nekrosen in den Blättern. Diese Symptome wurden quantifiziert und auf Signifikanz getestet.

b) Erysiphe cichoracearum

Der biotrophe Mehltau-Pilz wurde auf Arabidopsis-Pflanzen kultiviert. Zur Infektion der 4 Wochen alten transgenen *HvBgt1*-exprimierenden Arabidopsis-Pflanzen wurden mit einem feinen Pinsel Konidienträger auf der Oberfläche der Blätter abgenommen und auf die Blätter der transgenen Pflanzen gestrichen. Die Pflanzen wurden für 7 Tage bei 20˚C inkubiert. 7 Tage nach Inokulation wurden die Konidienträger auf den Blättern sichtbar, und es traten in den folgenden Tagen Chlorosen und Nekrosen zutage. Diese Symptome wurden quantifiziert und auf Signifikanz getestet.

c) Ergebnisse

Die transgenen Arabidopsis Pflanzen, die sense-Sequenzen für das *HvBgt1* expimieren, zeigen in den meisten Fällen sowohl gegen Peronospora parasitica als auch gegen Erysiphe cichoracearum eine signifikant erhöhte Resistenz im Vergleich zu nicht-transgenen Wildtyp-Pflanzen.

Die transgenen Arabidopsis Pflanzen, die antisense-Sequenzen für *AtBgt1* oder *HvBgt1* exprimieren, zeigen sowohl gegen Peronospora parasitica als auch gegen Erysiphe cichoracearum eine signifikant erhöhte Anfälligkeit im Vergleich zu nicht-transgenen Wildtyp-Pflanzen.

## Abbildungen

Abbildung 1: Darstellung des zeitlichen Verlaufs der Expression von *HvBgt1* bei der Nicht-Wirts- (Gerste (Ingrid) und *Bgt*) und der Wirts-Interaktion (Gerste (Ingrid) mit *Bgh*)

Dargestellt sind die in Tabelle 5 gezeigten relativen Expressionsdaten, mit der dazugehörenden Standardabweichung. Die Ergebnisse zeigen die Steigerung der Transkriptmenge bei der Nicht-Wirts-Interaktion im Verlauf der Messung.

## Patentansprüche

**1.** Verfahren zur Herstellung von transgenen Pflanzen bzw. Pflanzenzellen mit im Vergleich zu Wildtyp-Pflanzen bzw. Wildtyp-Pflanzenzellen erhöhter Pilzpathogenresistenz, **dadurch gekennzeichnet, dass** es die folgenden Schritte umfasst:

a) Herstellen eines rekombinanten Nukleinsäuremoleküls umfassend die folgenden Elemente in 5'-3'-Orientierung:

- regulatorische Sequenzen eines in Pflanzenzellen aktiven Promotors,
- operativ daran gebunden eine DNA-Sequenz ausgewählt aus der Gruppe bestehend aus:

i) DNA-Sequenzen umfassend Nukleotidsequenzen, die von SEQ ID No. 1 kodiert werden,
ii) DNA-Sequenzen umfassend Nukleotidsequenzen, die für Proteine mit der in SEQ ID No. 2 angegebenen Aminosäuresequenz kodieren, und/oder
iii) DNA-Sequenzen, die über die gesamte Sequenz zu der in SEQ ID No. 1 angegebenen Nukleotidsequenz eine Sequenzidentität von mindestens 80% aufweisen,
und die für ein Protein mit der Aktivität einer Peroxidase kodiert

b) Übertragen des rekombinanten Nukleinsäuremoleküls aus a) in Pflanzenzellen und Expression der DNA-Sequenzen, und
c) Regenerieren von Pflanzen aus den transformierten Pflanzenzellen wobei in Schritt b) die integration des rekombinanten Nukleinsäuremoleküls aus Schritt a) in das pflanzliche Genom erfolgt.

**2.** Isoliertes Nukleinsäuremolekül, enthaltend eine Nukleinsäuresequenz ausgewählt aus der Gruppe bestehend aus:

i) DNA-Sequenzen umfassend Nukleotidsequenzen, die von SEQ ID No. 1 kodiert werden,
ii) DNA-Sequenzen umfassend Nukleotidsequenzen, die für ein Protein mit der in SEQ ID No. 2 angegebenen Aminosäuresequenz kodieren, und/oder
iii) DNA-Sequenzen umfassend Nukleotidsequenzen, die über die gesamte Sequenz zu der in SEQ ID No. 1 angegebenen Nukleotidsequenz eine Sequenzidentität von mindestens 80% aufweisen,
und die für ein Protein mit der Aktivität einer Peroxidase kodiert.

**3.** Nukleinsäuremolekül nach Anspruch 2, wobei die Nukleinsäuresequenz aus *Hordeum vulgare* stammt.

**4.** Rekombinantes Peroxidase-Protein, kodiert durch eine Nukleinsäuresequenz wie in Anspruch 1 angegeben.

**5.** Rekombinantes Nukleinsäuremolekül, umfassend die folgenden Elemente in 5'-3'-Orientierung:

- regulatorische Sequenzen eines in Pflanzenzellen aktiven Promotors,
- operativ daran gebunden eine DNA-Sequenz wie in Anspruch 1 angegeben.

**6.** Rekombinantes Nukleinsäuremolekül nach Anspruch 5, weiterhin umfassend regulatorische Sequenzen, die in der Pflanzenzelle als Transkriptions-, Terminations- und/oder Polyadenylierungssignale dienen können und die operativ an die DNA-Sequenz nach Anspruch 1 gebunden sind.

**7.** Rekombinantes Nukleinsäuremolekül nach Anspruch 5,
**dadurch gekennzeichnet, dass** die DNA-Sequenz unter der Kontrolle eines konstitutiven Promotors exprimiert wird.

**8.** Rekombinantes Nukleinsäuremolekül nach Anspruch 7,
**dadurch gekennzeichnet, dass** es sich bei dem Promotor um den 35S CaMV- oder den Ubiquitinpromotor handelt.

**9.** Rekombinantes Nukleinsäuremolekül nach Anspruch 5,
**dadurch gekennzeichnet, dass** die DNA-Sequenz unter der Kontrolle eines gewebespezifischen Promotors exprimiert wird.

**10.** Rekombinantes Nukleinsäuremolekül nach Anspruch 9,
**dadurch gekennzeichnet, dass** der gewebespezifische Promotor ein Epidermis-, Mesophyll- oder Blatt-spezifischer Promotor ist.

**11.** Rekombinantes Nukleinsäuremolekül nach Anspruch 5,
**dadurch gekennzeichnet, dass** die DNA-Sequenz unter der Kontrolle eines induzierbaren Promotors exprimiertwird.

**12.** Rekombinantes Nukleinsäuremolekül nach Anspruch 11,
**dadurch gekennzeichnet, dass** der induzierbare Promotor ein pathogen- oder wundinduzierbarer Promotor ist.

**13.** Transgene Pflanzenzelle, enthaltend eine Nukleinsäuresequenz wie in Anspruch 1 beschrieben, wobei die Nukleinsäuresequenz in einer durch gentechnologische Methoden zustande gekommenen Expressionskassette vorliegt, in der sich entweder a) die Nukleinsäuresequenz oder b) eine mit der Nukleinsäuresequenz funktionell verknüpfte genetische Kontrollsequenz oder c) a) und b) nicht in ihrer natürlichen, genetischen Umgebung befindet.

**14.** Transgene Pflanzenzelle nach Anspruch 13, die einen gegenüber Wildtypzellen erhöhten Gehalt eines Proteins nach Anspruch 4 aufweist.

**15.** Transgene Pflanzenzelle nach Anspruch 13 oder 14, die eine gegenüber Wildtypzellen erhöhte Pilzepathogenresistenz aufweist.

**16.** Transgene Pflanzenzelle nach Anspruch 15, die eine erhöhte Resistenz gegenüber Mehltau-, Rost- und/oder Septoria-Pilzen aufweist.

**17.** Transgene Pflanzenzelle nach Anspruch 16, die eine erhöhte Resistenz gegenüber Formae speciales des Mehltaus aufweist.

**18.** Transgene Pflanze sowie transgene Teile dieser Pflanze, transgene Ernteprodukte und transgenes Vermehrungsmaterial dieser-Pflanze, wie Protoplasten, Pflahzenzellen, Kalli, Samen, Knollen, Stecklinge, sowie die transgenen Nachkommen dieser Pflanze enthaltend eine Pflanzenzelle nach einen der Ansprüche 13 bis 17.

**19.** Transgene Pflanze nach Anspruch 18,
**dadurch gekennzeichnet, dass** es sich bei der transgenen Pflanze um eine monokotyledone Pflanze handelt.

**20.** Transgene Pflanze nach Anspruch 19,
**dadurch gekennzeichnet, dass** es sich bei der monokotyledonen Pflanze um eine Pflanze, die zu den Gattungen Avena (Hafer), Triticum (Weizen), Secale (Roggen), Hordeum (Gerste), Oryza (Reis), Panicum, Pennisetum, Setaria, Sorghum (Hirse) oder Zea (Mais) gehört, handelt.

**21.** Transgene Pflanze nach Anspruch 18,
**dadurch gekennzeichnet, dass** es sich bei der transgenen Pflanze um eine dikotyledone Pflanze handelt.

**22.** Transgene Pflanze nach Anspruch 21,
**dadurch gekennzeichnet, dass** es sich bei der dikotyledonen Pflanze um Baumwolle, Leguminosen wie Hülsenfrüchte, Alfalfa, Sojabohne, Raps, Canola, Tomate, Zuckerrübe, Kartoffel, Zierpflanzen, Sonnenblume, Tabak oder Bäume handelt.

**23.** Verwendung einer DNA-Sequenz wie in Anspruch 1 angegeben zur Herstellung von transgenen Pflanzen und Pflanzenzellen mit im Vergleich zu Wildtyp-Pflanzen bzw. Wildtyp-Pflanzenzellen erhöhter Pilzpathogenresistenz.

**24.** Verwendung einer DNA-Sequenz wie in Anspruch 1 angegeben zur Identifizierung von Pflanzen, die eine Nicht-Wirts-Resistenz gegenüber Pilzpathogenen zeigen.

**25.** Verwendung einer DNA-Sequenz wie in Anspruch 1 angegeben als Marker für Nicht-Wirts-Resistenz in Getreide.

**Claims**

**1.** A method of generating transgenic plants or plant cells with a resistance to fungal pathogens which is increased in comparison with wild-type plants or wild-type plant cells, which comprises the following steps:

a) generating a recombinant nucleic acid molecule comprising the following elements in 5'-3' orientation:

- regulatory sequences of a promoter which is active in plant cells,
- in operable linkage thereto, a DNA sequence selected from the group consisting of:

i) DNA sequences comprising nucleotide sequences which are encoded by SEQ ID No. 1,
ii) DNA sequences comprising nucleotide sequences which code for proteins with the amino acid sequence specified in SEQ ID No. 2, and/or
iii) DNA sequences which have, over the entire sequence, a sequence identity of at least 80% to the nucleotide sequence specified in SEQ ID No. 1,
and coding for a protein with the activity of a peroxidise

b) transferring the recombinant nucleic acid molecule of a) into plant cells and expression of the DNA sequences, and
c) regenerating plants from the transformed plant cells,
where, in step b), the recombinant nucleic acid molecule of step a) is integrated into the plant genome.

**2.** An isolated nucleic acid molecule, comprising a nucleic acid sequence selected from the group consisting of:

i) DNA sequences comprising nucleotide sequences which are encoded by SEQ ID No. 1,
ii) DNA sequences comprising nucleotide sequences which code for a protein with the amino acid sequence specified in SEQ ID No. 2, and/or
iii) DNA sequences comprising nucleotide sequences which have, over the entire sequence, a sequence identity of at least 80% to the nucleotide sequence specified in SEQ ID No. 1,
and coding for a protein with the activity of a peroxidase.

**3.** The nucleic acid molecule according to claim 2, wherein the nucleic acid sequence originates from *Hordeum vulgare.*

**4.** A recombinant peroxidase protein, encoded by a nucleic acid sequence as specified in claim 1.

**5.** A recombinant nucleic acid molecule, comprising, in 5'-3' orientation, the following elements:

- regulatory sequences of a promoter which is active in plant cells,
- in operable linkage thereto, a DNA sequence as specified in claim 1.

**6.** The recombinant nucleic acid molecule according to claim 5, furthermore comprising regulatory sequences which can act as transcription, termination and/or polyadenylation signals in the plant cell and which are operably linked to the DNA sequence according to claim 1.

**7.** The recombinant nucleic acid molecule according to claim 5, **wherein** the DNA sequence is expressed under the control of a constitutive promoter.

**8.** The recombinant nucleic acid molecule according to claim 7, **wherein** the promoter is the 35S CaMV promoter or the ubiquitin promoter.

**9.** The recombinant nucleic acid molecule according to claim 5, wherein the DNA sequence is expressed under the control of a tissue-specific promoter.

**10.** The recombinant nucleic acid molecule according to claim 9, **wherein** the tissue-specific promoter is an epidermis-, mesophyll- or leaf-specific promoter.

**11.** The recombinant nucleic acid molecule according to claim 5, **wherein** the DNA sequence is expressed under the control of an inducible promoter.

**12.** The recombinant nucleic acid molecule according to claim 11, **wherein** the inducible promoter is a pathogen- or wound-inducible promoter.

**13.** A transgenic plant cell, comprising a nucleic acid sequence as described in claim 1, where the nucleic acid sequence is present in an expression cassette created by recombinant methods, in which cassette either a) the nucleic acid sequence or b) a genetic control sequence which is functionally linked to the nucleic acid sequence or c) a) and b) is/are not in their natural genetic environment.

**14.** The transgenic plant cell according to claim 13, which has a content of a protein according to claim 4 which is increased in comparison with wild-type cells.

**15.** The transgenic plant cell according to claim 13 or 14, which has a resistance to fungal pathogens which is increased in comparison with wild-type cells.

**16.** The transgenic plant cell according to claim 15, which has an increased resistance to mildew, rust and/or septoria fungi.

**17.** The transgenic plant cell according to claim 16, which has an increased resistance to formae speciales of mildew.

**18.** A transgenic plant and transgenic parts of this plant, transgenic crop products and transgenic propagation material of this plant, such as protoplasts, plant cells, kalli, seeds, tubers, cuttings, and the transgenic progeny of this plant, comprising a plant cell according to any of claims 13 to 17.

**19.** The transgenic plant according to claim 18, **wherein** the transgenic plant is a monocotyledonous plant.

**20.** The transgenic plant according to claim 19, **wherein** the monocotyledonous plant is a plant which belongs to the genera Avena (oats), Triticum (wheat), Secale (rye), Hordeum (barley), Oryza (rice), Panicum, Pennisetum, Setaria, Sorghum (sorgo) or Zea (maize).

**21.** The transgenic plant according to claim 18, **wherein** the transgenic plant is a dicotyledonous plant.

**22.** The transgenic plant according to claim 21, **wherein** the dicotyledonous plant is cotton, legumes such as pulses, alfalfa, soybean, oilseed rape, canola, tomato, sugar beet, potato, ornamentals, sunflower, tobacco or trees.

**23.** The use of a DNA sequence as specified in claim 1 for the generation of transgenic plants and plant cells which have a resistance to fungal pathogens which is increased in comparison with wild-type plants or wild-type plant cells.

**24.** The use of a DNA sequence as specified in claim 1 for identifying plants which exhibit a non-host resistance to fungal pathogens.

**25.** The use of a DNA sequence as specified in claim 1 as a marker for non-host resistance in cereals.

**Revendications**

**1.** Procédé de préparation de plantes ou de cellules végétales transgéniques, présentant une résistance aux champignons pathogènes accrue par comparaison avec les plantes de type sauvage ou les cellules végétales de type sauvage, **caractérisé en ce qu'**il comprend les étapes suivantes :

a) préparation d'une molécule d'acide nucléique recombinante, comprenant les éléments suivants, dans le sens 5'-3' :

- des séquences régulatrices d'un promoteur actif dans les cellules végétales,
- liée d'une manière opérationnelle à ce dernier, une séquence d'ADN choisie dans le groupe consistant en :

i) les séquences d'ADN comprenant des séquences nucléotidiques qui sont codées par SEQ ID N° 1,
ii) les séquences d'ADN comprenant des séquences nucléotidiques qui codent pour des protéines ayant la séquence d'acides aminés présentée dans SEQ ID N° 2, et/ou
iii) les séquences d'ADN qui, sur la totalité de leur séquence, présentent avec la séquence- nucléotidique présentée dans SEQ ID N° 1 une identité de séquence d'au moins 80 %,
et qui code pour une protéine ayant l'activité d'une peroxydase,

b) transfert de la molécule d'acide nucléique recombinante de a) dans des cellules végétales et expression des séquences d'ADN, et
c) régénération de plantes à partir des cellules végétales transformées,
l'intégration de la molécule d'acide nucléique recombinante de l'étape a) étant, dans l'étape b), réalisée dans le génome végétal.

**2.** Molécule d'acide nucléique isolée, contenant une séquence d'acides nucléiques choisie dans le groupe consistant en :

i) les séquences d'ADN comprenant des séquences nucléotidiques qui sont codées par SEQ ID N° 1,
ii) les séquences d'ADN comprenant des séquences nucléotidiques qui codent pour une protéine ayant la séquence d'acides aminés présentée dans SEQ ID N° 2, et/ou
iii) les séquences d'ADN comprenant des séquences nucléotidiques qui, sur la totalité de leur séquence, présentent avec la séquence nucléotidique présentée dans SEQ ID N°1 une identité de séquence d'au moins 80 %, et qui code pour une protéine ayant l'activité d'une peroxydase.

3. Molécule d'acide nucléique selon la revendication 2, dans laquelle la séquence d'acides nucléiques provient de *Hordeum vulgaire*.

4. Protéine peroxydase recombinante, codée par une séquence d'acides nucléiques telle qu'indiquée dans la revendication 1.

5. Molécule d'acide nucléique recombinante, comprenant les éléments suivants, dans le sens 5'-3' :

   - des séquences régulatrices d'un promoteur actif dans les cellules végétales,
   - liée à ce dernier d'une manière opérationnelle, une séquence d'ADN telle qu'indiquée dans la revendication 1.

6. Molécule d'acide nucléique recombinante selon la revendication 5, qui comprend en outre des séquences régulatrices, qui dans la cellule végétale peuvent servir de signaux de transcription, de terminaison et/ou de polyadénylation, et qui sont liées d'une manière opérationnelle à la séquence d'ADN selon la revendication 1.

7. Molécule d'acide nucléique recombinante selon la revendication 5, **caractérisée en ce que** la séquence d'ADN est exprimée sous le contrôle d'un promoteur constitutif.

8. Molécule d'acide nucléique recombinante selon la revendication 7, **caractérisée en ce que**, pour ce qui concerne le promoteur, il s'agit du promoteur 35S du CaMV ou du promoteur de l'ubiquitine.

9. Molécule d'acide nucléique recombinante selon la revendication 5, **caractérisée en ce que** la séquence d'ADN est exprimée sous le contrôle d'un promoteur spécifique de tissu.

10. Molécule d'acide nucléique recombinante selon la revendication 9, **caractérisée en ce que** le promoteur spécifique de tissu est un promoteur spécifique d'épiderme, de mésophylle ou de feuille.

11. Molécule d'acide nucléique recombinante selon la revendication 5, **caractérisée en ce que** la séquence d'ADN est exprimée sous le contrôle d'un promoteur inductible.

12. Molécule d'acide nucléique recombinante selon la revendication 11, **caractérisée en ce que** le promoteur inductible est un promoteur inductible par un micro-organisme pathogène ou une blessure.

13. Cellule végétale transgénique, contenant une séquence d'acides nucléiques telle que décrite dans la revendication 1, la séquence d'acides nucléiques étant présente dans une cassette d'expression réalisée par une méthode de génie génétique, cassette dans laquelle a) la séquence d'acides nucléiques, ou b) une séquence de contrôle génétique liée d'une manière fonctionnelle à la séquence d'acides nucléiques, ou c) a) et b), ne se trouvent pas dans leur environnement génétique naturel.

14. Cellule végétale transgénique selon la revendication 13, qui présente une teneur en une protéine selon la revendication 4 qui est accrue par rapport aux cellules de type sauvage.

15. Cellule végétale transgénique selon la revendication 13 ou 14, qui présente une résistance aux champignons pathogènes qui est accrue par comparaison avec les cellules de type sauvage.

16. Cellule végétale transgénique selon la revendication 15, qui présente une résistance accrue aux champignons responsables du mildiou, de la rouille et/ou de la septoriose.

17. Cellule végétale transgénique selon la revendication 16, qui présente une résistance accrue à des formes spéciales du mildiou.

18. Plante transgénique, ainsi que parties transgéniques de cette plante, produits de récolte transgéniques et matériel transgénique de cette plante, tel que les protoplastes, les cellules végétales, le cal, les graines, les tubercules, les boutures, ainsi que la descendance transgénique de cette plante, contenant une cellule végétale selon l'une des revendications 13 à 17.

19. Plante transgénique selon la revendication 18, **caractérisée en ce que**, pour ce qui concerne la plante transgénique, il s'agit d'une plante monocotylédone.

20. Plante transgénique selon la revendication 19, **caractérisée en ce que**, pour ce qui concerne la plante monocotylédone, il s'agit d'une plante qui appartient aux genres Avena (avoine), Triticum (blé), Secale (seigle), Hordeum (orge), Oryza (riz), Panicum, Pennisetum, Setaria, Sorghum (millet) ou Zea (maïs).

21. Plante transgénique selon la revendication 18, **caractérisée en ce que**, pour ce qui concerne la plante transgénique, il s'agit d'une plante dicotylédone.

22. Plante transgénique selon la revendication 21, **caractérisée en ce que**, pour ce qui concerne la plante dicotylédone, il s'agit du coton, de légumineuses telles que les légumineuses, la luzerne, le soja, le colza, le canola, la tomate, la betterave à sucre, la pomme de terre, les plantes ornementales, le tournesol, le tabac ou les arbres.

23. Utilisation d'une séquence d'ADN telle que présentée dans la revendication 1 pour la fabrication de plantes transgéniques et de cellules végétales présentant une résistance aux champignons pathogènes accrue par comparaison avec les plantes de type sauvage ou les cellules végétales de type sauvage.

24. Utilisation d'une séquence d'ADN telle qu'indiquée dans la revendication 1 pour identifier des plantes qui présentent une résistance non-hôte à des champignons pathogènes.

25. Utilisation d'une séquence d'ADN telle qu'indiquée dans la revendication 1 en tant que marqueur de résistance non-hôte dans les céréales.

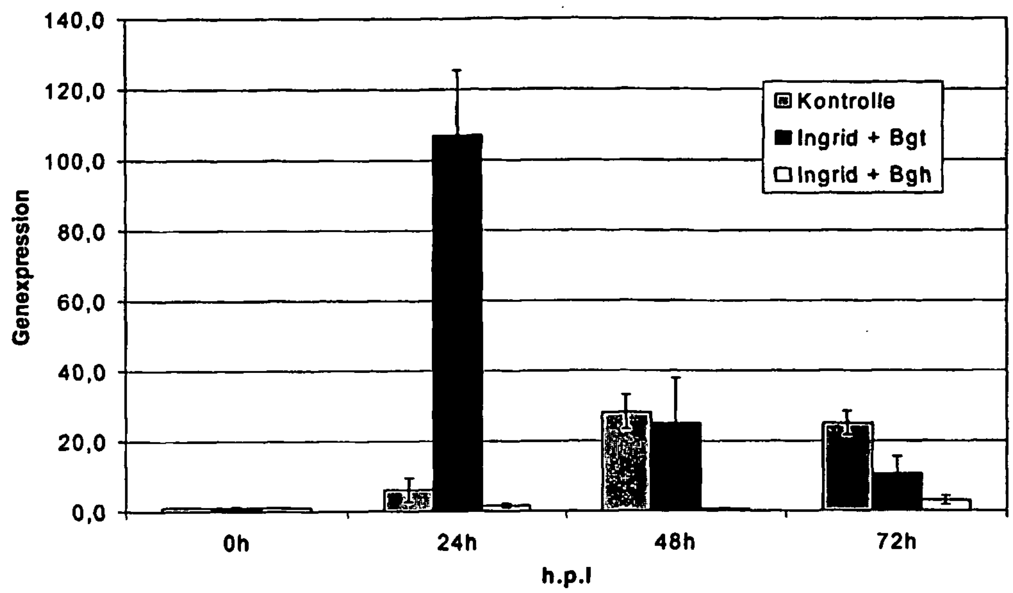
140,0
120,0
100,0
80,0
60,0
40,0
20,0
0,0
Genexpression
Kontrolle
Ingrid + Bgt
Ingrid + Bgh
0h
24h
48h
72h
h.p.I

Abb. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0207023 A
- US 5565350 A
- WO 0015815 A
- WO 9606166 A
- WO 9943838 A
- US 6072050 A
- US 5659026 A
- US 5428148 A
- WO 03093483 A
- US 5814618 A
- US 5789156 A
- EP 120515 A
- US 6137030 A


### In der Beschreibung aufgeführte Nicht-Patentliteratur


- **Schopfer ; Brennicke.** Pflanzenphysiologie. Springer Verlag, 1999
- **Flor.** *Annu. Rev. Phytopathology,* 1971, vol. 9, 275 296
- **Keen.** *Annu. Rev. Genet.,* 1992, vol. 24, 447-463
- **Staskawicz et al.** *Science,* 1995, vol. 268, 661-667
- **Neu et al.** *American Cytopathol. Society, MPMI,* 2003, vol. 16 (7), 626-633
- **Heath.** *Can. J. Plant Pathol.,* 2002, vol. 24, 259-264
- **Heath.** *Phytopathology,* 1981, vol. 71, 1121-1123
- **Heath.** *Physiol. Mol. Plant Pathol.,* 2001, vol. 58, 53-54
- **Kamoun et al.** *Plant Cell,* 1998, vol. 10, 1413-1425
- **Lauge et al.** *Plant J.,* 2000, vol. 23, 735-745
- **Whalen et al.** *Proc. Natl. Acad. Sci. USA,* 1988, vol. 85, 6743-6747
- **Ferrari et al.** *The Plant Journal,* 2003, vol. 35, 193-205
- **Collins et al.** The Powdery Mildew Accomprehensive Treatise. American Phytopathological Society, 2002
- **Peterhänsel et al.** *Plant Cell,* 1997, vol. 9, 1397-1409
- **Schulze-Lefert ; Vogel.** *Trends in Plant Science,* 2000, vol. 5, 343-348
- **Chance ; Maehley.** *Method Enzymol.,* 1955, vol. 11, 764-775
- **Pandolfini et al.** *Plant Cell Environ,* 1992, vol. 15, 719-725
- **Chirgwin et al.** *Biochemistry,* 1979, vol. 18, 5294-5299
- **Sambrook ; Russell.** Molecular Cloning: A Laboratory Manual. Cold Spring Harbour Laboratory Press, 2001
- Lehrbüchern Current Protocols in Molecular Biology. John Wiley & Sons, 1989
- Nucleic Acids Hybridization: A Practical Approach. IRL Press at Oxford University Press, 1985
- Essential Molecular Biology: A Practical Approach. IRL Press at Oxford University Press, 1991
- **Feng ; Doolittle.** *J. Mol. Evolution,* 1987, vol. 25, 351-360
- **Higgins et al.** *CABIOS,* 1989, vol. 5, 151-153
- **Needleman ; Wunsch.** *J. Mol. Biol.,* 1970, vol. 48, 443-453
- **Smith ; Waterman.** *Adv. Appl. Math.,* 1981, vol. 2, 482-489
- **Odell et al.** *Nature,* 1985, vol. 313, 810-812
- **McElroy et al.** *Plant Cell,* 1990, vol. 2, 163-171
- **Christensen et al.** *Plant Mol. Biol.,* 1989, vol. 12, 619-632
- **Christensen et al.** *Plant Mol. Biol.,* 1992, vol. 18, 675-689
- **Last et al.** *Theor. Appl. Genet.,* 1991, vol. 81, 581-588
- **Velten et al.** *EMBO J.,* 1984, vol. 3, 2723-2730
- **Redolfi et al.** *Neth. J. Plant Pathol.,* 1983, vol. 89, 245-254
- **Uknes et al.** *Plant Cell,* 1992, vol. 4, 645-656
- **Van Loon.** *Plant Mol. Virol.,* 1985, vol. 4, 111-116
- **Marineau et al.** *Plant Mol. Biol.,* 1987, vol. 9, 335-342
- **Matton et al.** *Molecular Plant-Microbe Interactions,* 1989, vol. 2, 325-331
- **Somsisch et al.** *Proc. Natl. Acad. Sci. USA,* 1986, vol. 83, 2427-2430

- **Somsisch et al.** *Mol. Gen. Genet.,* 1988, vol. 2, 93-98
- **Yang.** *Proc. Natl. Acad. Sci. USA,* 1996, vol. 93, 14972-14977
- **Chen et al.** *Plant J.,* 1996, vol. 10, 955-966
- **Zhang et al.** *Proc. Natl. Acad. Sci. USA,* 1994, vol. 91, 2507-2511
- **Warner et al.** *Plant J.,* 1993, vol. 3, 191-201
- **Siebertz et al.** *Plant Cell,* 1989, vol. 1, 961-968
- **Ryan.** *Ann. Rev. Phytopathol.,* 1990, vol. 28, 425-449
- **Duan et al.** *Nature Biotechnology,* 1996, vol. 14, 494-498
- **Stanford et al.** *Mol. Gen. Genet.,* 1989, vol. 215, 200-208
- **McGurl et al.** *Science,* 1992, vol. 225, 1570-1573
- **Rohmeier et al.** *Plant Mol. Biol.,* 1993, vol. 22, 783-792
- **Eckelkamp et al.** *FEBS Letters,* 1993, vol. 323, 73-76
- **Corderok et al.** *Plant J.,* 1994, vol. 6 (2), 141-150
- **Vaghchhipawala et al.** *Genome,* 2004, vol. 47 (2), 404-413
- **Kaothien et al.** *Plant Mol. Biol.,* 2002, vol. 49 (6), 591-599
- **Ito et al.** *Plant Science,* 2000, vol. 155 (1), 85-100
- **Nishiuchi et al.** *Plant Physiol.,* 1999, vol. 121 (4), 1239-1246
- **Gatz.** *Annu. Rev. Plant Physiol. Plant Mol. Biol.,* 1997, vol. 48, 89-108
- **Schena et al.** *Proc. Natl. Acad. Sci. USA,* 1991, vol. 88, 10421-10425
- **McNellis et al.** *Plant J.,* 1998, vol. 14 (2), 247-257
- **Gatz et al.** *Mol. Gen. Genet.,* 1991, vol. 227, 229-237
- **Yamamoto et al.** *Plant J.,* 1997, vol. 1 (2), 255-265
- **Kwon et al.** *Plant Physiol,* 1994, vol. 105, 357-367
- **Yamamoto et al.** *Plant Cell Physiol.,* 1994, vol. 35 (5), 773-778
- **Gotor et al.** *Plant J.,* 1993, vol. 3, 509-518
- **Orozco et al.** *Plant Mol. Biol.,* 1993, vol. 23 (6), 1129-1138
- **Matsuoka et al.** *Proc. Natl. Acad. Sci. USA,* 1993, vol. 90 (20), 9586-9590
- **Stockhaus et al.** *Proc. Natl. Acad. G. USA,* 1987, vol. 84, 7943-7947
- **Stockhaus et al.** *EMBO J.,* 1989, vol. 8, 2445-2451
- **Hooker et al.** *Plant Physiol.,* 2002, vol. 129 (4), 1568-1580
- **Kunst et al.** *Biochem. Soc. Trans.,* 2000, vol. 28 (6), 651-654
- **Nicholass et al.** *Plant Mol. Biol.,* 1995, vol. 28, 423-435
- **Atkinson et al.** *Plant Mol. Biol.,* 1998, vol. 38, 449-460
- **van Haaren et al.** *Plant Mol. Biol.,* 1991, vol. 17, 615-630
- **Kyozuka et al.** *Plant Physiol.,* 1993, vol. 102 (3), 991-1000
- **Kausch et al.** *Plant Mol Biol.,* 2001, vol. 45 (1), 1-15
- **Thain et al.** *Plant Physiol.,* 2002, vol. 130, 102-110
- **Kagaya et al.** *Mol Gen Genet.,* 1995, vol. 248 (6), 668-674
- **Goeddel.** Gene Expression Technology: Methods in Enzymology. Academic Press, 1990, vol. 185
- **Gruber ; Crosby.** Methods in Plant Molecular Biology and Biotechnology. CRC Press, 89-108
- Cloning Vectors. Elsevier, 1985
- **Falciatore et al.** *Marine Biotechnology,* 1999, vol. 1 (3), 239-251
- **Becker, D. ; Kemper, E. ; Schell, J. ; Masterson, R.** Plant Mol. Biol. 1992, vol. 20, 1195-1197
- **Bevan, M.W.** *Nucl. Acids Res.,* 1984, vol. 12, 8711-8721
- Vectors for Gene Transfer in Higher Plants; in: Transgenic Plants, Bd. 1, Engineering and Utilization. Academic Press, 1993, vol. 1, 15-38
- **Gallie et al.** *Nucl. Acids Research,* 1987, vol. 15, 8693-8711
- **Kermode.** *Crit. Rev. Plant Sci.,* 1996, vol. 15 (4), 285-423
- **Hellens et al.** *Trends in Plant Science,* 2000, vol. 5, 446-451
- Plant Molecular Biology and Biotechnology. CRC Press, 1993, 71-119
- **F.F. White.** Vectors for Gene Transfer in Higher Plants; in: Transgenic Plants, Bd. 1, Engineering and Utilization. Academic Press, 1993, vol. 1, 15-38
- **B. Jenes et al.** *Techniques for Gene Transfer, in: Transgenic Plants, Bd. 1, Engineering and Utilization,* 1993, vol. 1, 128-143
- **Potrykus.** *Annu. Rev. Plant Physiol. Plant Molec. Biol.,* 1991, vol. 42, 205-225
- **Holsters et al.** *Molecular and General Genetics,* 1978, vol. 163, 181-187
- **L. Willmitzer et al.** Transgenic Plants in: Biotechnology, A Multi-Volume Comprehensive Treatise. VCH Weinheim, 1993, vol. 2, 627-659
- **Chan et al.** *Plant Mol. Biol.,* 1993, vol. 22, 491-506
- **Wan ; Lemaux.** *Plant Physiol.,* 1994, vol. 104, 37-48
- **Vasil et al.** *Bio/Technology,* 1993, vol. 11, 1553-1558

- **Ritala et al.** *Plant Mol. Biol.,* 1994, vol. 24, 317-325
- **Spencer et al.** *Theor. Appl. Genet.,* 1990, vol. 79, 625-631
- **McCormick et al.** *Plant Cell Reports,* 1986, vol. 5, 81-84
- **Dunwell JM.** *J Exp Bot.,* 2000, vol. 51, 487-496
- **Sanger et al.** *Proc. Natl. Acad. Sci. USA,* 1977, vol. 74, 5463-5467
- **Radelof et al.** *Nucleic Acids Res.,* 1998, vol. 26 (23), 5358-64
- **Schweizer P et al.** *Mol Plant Microbe Interact,* 1999, vol. 12, 647-54
- **Steve Clough ; Andrew Bent.** *Plant J,* 1998, vol. 16 (6), 735-743
- **Bechtold N et al.** *CR Acad Sci Paris, Life Sciences,* 1993, vol. 316, 1194-1199

SEQUENCE LISTING

<110> BASF Plant Science GmbH

<120> Verfahren zur Erhöhung der Pathogenresistenz in transgenen Pflanzen durch Expression einer Peroxidase

<130> B 7661

<160> 12

<170> PatentIn version 3.3

<210> 1
<211> 945
<212> DNA
<213> Hordeum vulgare

<400> 1

```
atggcctcta cttcgtccct atcagtggtg ttgctcttgt gcctggccgt ggcggcgtcg      60
gcgcagctgt cgccgacgtt ctaccaaacg acgtgcccga acgctctgtc caccatcaag     120
gccgccgtga cggccgccgt gaacaatgag aaccgcatgg gcgcgtcgct gctccggctg     180
cacttccacg actgcttcgt ccaaggttgt gacgcgtctg ttctgctgtc tggcatggaa     240
caaaacgcgg cgccgaacgt catgtccctg cgaggcttcg aagtcataga cagcatcaag     300
gcgaagctcg agaccatgtg caagcagacc gtctcctgcg ccgacatcct caccgtcgct     360
gcccgcgatt ccgtcgtcgc cttgggaggg ccatcgtgga cggttccgct aggaaggagg     420
gactccacca atgcaaacga agcagcggcg aactccgacc tacctccccc gttcttcgac     480
ctcgtcaacc tcacccaatc cttcggcgac aagggcttca ccgtcaccga catggtcgcg     540
ctctccggtg cccacaccat cggacaggcg cagtgccaga acttcaggga taggctctac     600
aacgagacta acatcaactc cggcttcgcg acgtcgctca aggccaactg cccccggccg     660
accggctccg gcgaccgcaa cctggccaat ctggacgtgt ctaccccgta ctcattcgac     720
aacgcctact acagcaacct caagtcccag aaggggctcc tgcactctga ccaggtgctc     780
ttcaccggca cgggcggcgg cacggacaac atcgtcaaca acttcgcgag caacccagct     840
gcgttcagcg gcgcctttgc ctcggccatg gtgaagatgg ggaacctcag cccattgact     900
ggctctcagg ggcaggtcag gctgagctgc tccaaggtga attaa                     945
```

<210> 2

<211> 314
<212> PRT
<213> Hordeum vulgare

<400> 2

```
Met Ala Ser Thr Ser Ser Leu Ser Val Val Leu Leu Leu Cys Leu Ala
1               5                   10                  15
```

```
Val Ala Ala Ser Ala Gln Leu Ser Pro Thr Phe Tyr Gln Thr Thr Cys
            20                  25                  30

Pro Asn Ala Leu Ser Thr Ile Lys Ala Ala Val Thr Ala Ala Val Asn
        35                  40                  45

Asn Glu Asn Arg Met Gly Ala Ser Leu Leu Arg Leu His Phe His Asp
    50                  55                  60

Cys Phe Val Gln Gly Cys Asp Ala Ser Val Leu Leu Ser Gly Met Glu
65                  70                  75                  80

Gln Asn Ala Ala Pro Asn Val Met Ser Leu Arg Gly Phe Glu Val Ile
                85                  90                  95

Asp Ser Ile Lys Ala Lys Leu Glu Thr Met Cys Lys Gln Thr Val Ser
            100                 105                 110

Cys Ala Asp Ile Leu Thr Val Ala Ala Arg Asp Ser Val Val Ala Leu
        115                 120                 125

Gly Gly Pro Ser Trp Thr Val Pro Leu Gly Arg Arg Asp Ser Thr Asn
    130                 135                 140

Ala Asn Glu Ala Ala Ala Asn Ser Asp Leu Pro Pro Pro Phe Phe Asp
145                 150                 155                 160

Leu Val Asn Leu Thr Gln Ser Phe Gly Asp Lys Gly Phe Thr Val Thr
                165                 170                 175

Asp Met Val Ala Leu Ser Gly Ala His Thr Ile Gly Gln Ala Gln Cys
            180                 185                 190

Gln Asn Phe Arg Asp Arg Leu Tyr Asn Glu Thr Asn Ile Asn Ser Gly
        195                 200                 205

Phe Ala Thr Ser Leu Lys Ala Asn Cys Pro Arg Pro Thr Gly Ser Gly
    210                 215                 220

Asp Arg Asn Leu Ala Asn Leu Asp Val Ser Thr Pro Tyr Ser Phe Asp
225                 230                 235                 240

Asn Ala Tyr Tyr Ser Asn Leu Lys Ser Gln Lys Gly Leu Leu His Ser
                245                 250                 255

Asp Gln Val Leu Phe Thr Gly Thr Gly Gly Gly Thr Asp Asn Ile Val
            260                 265                 270
```

```
Asn Asn Phe Ala Ser Asn Pro Ala Ala Phe Ser Gly Ala Phe Ala Ser
        275                 280                 285

Ala Met Val Lys Met Gly Asn Leu Ser Pro Leu Thr Gly Ser Gln Gly
    290                 295                 300

Gln Val Arg Leu Ser Cys Ser Lys Val Asn
305                 310
```

<210> 3
<211> 951
<212> DNA
<213> Arabidopsis thaliana

<400> 3

```
atgttaaagg tggtgttgtt gatgatgata atgatgttgg cgtcacagtc cgaggctcag      60
ctgaaccgtg acttttacaa ggaaagctgt ccatcattgt tccttgtcgt gagacgagtc     120
gtgaaacggg ccgtggccag agagcctcgc atgggtgctt ctctccttcg tttgttcttc     180
catgattgtt ttgtcaatgg gtgtgacgga tccttactgt tggatgacac accgtctttt     240
ttgggagaga aaacctcagg acccagcaat aactctgtga gggggttcga agtgatcgac     300
aaaatcaagt ttaaggttga gaaaatgtgc ccgggcatcg tctcatgcgc agacattcta     360
gccatcactg ctcgggactc cgttctcctc ctaggtggac cggggtggag cgtgaaactt     420
ggaagaagag actctacgac ggcgaacttc gcggccgcga actccggagt catccctcct     480
ccgatcacta cccttagcaa cctcataaac cgtttcaaag cacaaggttt gtccacacgt     540
gacatggtcg ccctctctgg tgctcacacc attggacgag cccaatgtgt tacattcaga     600
aaccgaatct acaacgcaag caatatcgac acctctttcg ccatctctaa acggaggaac     660
tgtcctgcca ccagtggctc cggagacaac aagaaagcca atcttgacgt ccgctctccc     720
gataggttcg accacggctt ctacaagcaa cttctgagca aaaaaggttt gcttacgtca     780
gaccaagtcc tctttaataa tggtcctacc gactcgctcg tcatagctta cagccacaat     840
ctcaatgcct tctaccgcga ctttgcaagg gcaatgatta agatgggaga catcagcccc     900
ctcaccggat ccaatggtca gatccgccaa aactgtcgga ggcccaactg a              951
```

<210> 4
<211> 316
<212> PRT
<213> Arabidopsis thaliana

<400> 4

```
Met Leu Lys Val Val Leu Leu Met Met Ile Met Met Leu Ala Ser Gln
1               5                   10                  15
```

```
Ser Glu Ala Gln Leu Asn Arg Asp Phe Tyr Lys Glu Ser Cys Pro Ser
            20                  25                  30

Leu Phe Leu Val Val Arg Arg Val Val Lys Arg Ala Val Ala Arg Glu
        35                  40                  45

Pro Arg Met Gly Ala Ser Leu Leu Arg Leu Phe Phe His Asp Cys Phe
    50                  55                  60

Val Asn Gly Cys Asp Gly Ser Leu Leu Leu Asp Asp Thr Pro Ser Phe
65                  70                  75                  80

Leu Gly Glu Lys Thr Ser Gly Pro Ser Asn Asn Ser Val Arg Gly Phe
                85                  90                  95

Glu Val Ile Asp Lys Ile Lys Phe Lys Val Glu Lys Met Cys Pro Gly
            100                 105                 110

Ile Val Ser Cys Ala Asp Ile Leu Ala Ile Thr Ala Arg Asp Ser Val
        115                 120                 125

Leu Leu Leu Gly Gly Pro Gly Trp Ser Val Lys Leu Gly Arg Arg Asp
    130                 135                 140

Ser Thr Thr Ala Asn Phe Ala Ala Ala Asn Ser Gly Val Ile Pro Pro
145                 150                 155                 160

Pro Ile Thr Thr Leu Ser Asn Leu Ile Asn Arg Phe Lys Ala Gln Gly
                165                 170                 175

Leu Ser Thr Arg Asp Met Val Ala Leu Ser Gly Ala His Thr Ile Gly
            180                 185                 190

Arg Ala Gln Cys Val Thr Phe Arg Asn Arg Ile Tyr Asn Ala Ser Asn
        195                 200                 205

Ile Asp Thr Ser Phe Ala Ile Ser Lys Arg Arg Asn Cys Pro Ala Thr
    210                 215                 220

Ser Gly Ser Gly Asp Asn Lys Lys Ala Asn Leu Asp Val Arg Ser Pro
225                 230                 235                 240

Asp Arg Phe Asp His Gly Phe Tyr Lys Gln Leu Leu Ser Lys Lys Gly
                245                 250                 255

Leu Leu Thr Ser Asp Gln Val Leu Phe Asn Asn Gly Pro Thr Asp Ser
            260                 265                 270
```

```
Leu Val Ile Ala Tyr Ser His Asn Leu Asn Ala Phe Tyr Arg Asp Phe
        275                 280                 285

Ala Arg Ala Met Ile Lys Met Gly Asp Ile Ser Pro Leu Thr Gly Ser
    290                 295                 300

Asn Gly Gln Ile Arg Gln Asn Cys Arg Arg Pro Asn
305                 310                 315
```

<210> 5
<211> 27
<212> DNA
<213> Artificial

<220>
<223> Primer

<400> 5
agaagtatgt taaaggtggt gttgttg 27

<210> 6
<211> 23
<212> DNA
<213> Artificial

<220>
<223> Primer

<400> 6
tgacgaagag gtgattattg agc 23

<210> 7
<211> 21
<212> DNA
<213> Artificial

<220>
<223> Primer

<400> 7
cgaggccctt gtgacataca t 21

<210> 8
<211> 25
<212> DNA
<213> Artificial

<220>
<223> Primer

<400> 8
acttaggtgt cgttacttgg accat 25

<210> 9

<211> 20
<212> DNA
<213> Artificial

<220>
<223> Primer

<400> 9
cgtccctgcc ctttgtacac 20

<210> 10
<211> 21
<212> DNA
<213> Artificial

<220>
<223> Primer

<400> 10
aacacttcac cggaccattc a 21

<210> 11
<211> 26
<212> DNA
<213> Artificial

<220>
<223> Primer

<400> 11
gcttattcag cagccaacaa agtaac 26

<210> 12
<211> 28
<212> DNA
<213> Artificial

<220>
<223> Primer

<400> 12
ctgtttcaca agtttatggt ccaagtaa 28